(19) Europäisches Patentamt
European Patent Office
Office européen des brevets

(11) EP 1 954 868 B1

(12) EUROPEAN PATENT SPECIFICATION

(45) Date of publication and mention of the grant of the patent:
**14.03.2012 Bulletin 2012/11**

(21) Application number: **06811651.6**

(22) Date of filing: **05.10.2006**

(51) Int Cl.:
***D06M 13/513*** *(2006.01)*
***D06M 15/643*** *(2006.01)*
***D06M 13/51*** *(2006.01)*
***C08G 77/16*** *(2006.01)*
***D21H 19/32*** *(2006.01)*

(86) International application number:
**PCT/JP2006/320354**

(87) International publication number:
**WO 2007/043599 (19.04.2007 Gazette 2007/16)**

(54) **FIBER-TREATING AGENT**

FASERBEHANDLUNGSMITTEL

AGENT DE TRAITEMENT DE FIBRES

(84) Designated Contracting States:
**DE FR GB**

(30) Priority: **07.10.2005 JP 2005295140**

(43) Date of publication of application:
**13.08.2008 Bulletin 2008/33**

(73) Proprietor: **Kao Corporation**
**Chuo-ku**
**Tokyo 103-8210 (JP)**

(72) Inventors:
- **SUZUKI, Keigo**
  **Wakayama-shi, Wakayama 6408580 (JP)**
- **MIYANAGA, Seiichi**
  **Sumida-ku, Tokyo 1318501 (JP)**

(74) Representative: **HOFFMANN EITLE**
**Patent- und Rechtsanwälte**
**Arabellastraße 4**
**81925 München (DE)**

(56) References cited:
**EP-A1- 0 535 649**
**US-A- 4 170 690**
**US-A- 4 517 375**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

Field of the invention

The present invention relates to a fiber-treating agent, a method of producing the same, a method of treating fibers with the same, and use of the fiber-treating agent to treat fibers.

Related arts to the invention

For the purpose of conferring water repellency, toughness, and flame retardancy on fibers, the treatment of fibers with a silicon compound has been conducted. For example, fibers can be endowed with water repellency to reduce the water content of the fibers thereby giving quick-drying properties to the fibers and clothes. That is, drying after washing is a time-consuming step, and it is a conventionally pursued problem to reduce the drying time. Especially in the rainy season and in winter, indoor drying is often conducted, and there are increasing cases where for pollinosis control measures, laundry cannot be dried outdoor. Under these circumstances, there is stronger demand for reduction in drying time.

For achieving this object, a method of reducing the water content of fibers by hydrophobation has been examined. JP-A 2003-342875 discloses a method of processing cellulose fibers, which contains treating cellulose fibers with an alkali metal hydroxide, washing them with water, and treating them with a hydrophobating processing agent such as a resin processing agent, a hydrophobating crosslinking agent or a hydrophobating agent.

From the viewpoint of pursuing quick-drying properties and water absorption, JP-A 2005-89882 discloses a water-absorbing quick-drying-conferring composition containing a copolymer of a specific monomer containing a silicone-containing monomer and an organic solvent, and discloses a method of treating fibers by spraying a silicone-containing copolymer onto objective fibers and then evaporating a solvent.

On one hand, JP-A 2002-61094 discloses, as a method of conferring toughness on fibers, a method of coating fibers which contains coating a fiber material such as paper or cloth with a silane-based coating solution containing an alkoxysilane condensate as a major component, then curing and hardening it by the action of a catalyst to form a surface thereon.

As an example of conferring water repellency or oil repellency on fibers, JP-A 9-249748 discloses a fiber-treating agent wherein a reaction product obtained by cohydrolysis/condensation of alkyl fluoride group-containing alkoxysilane, alkyl group-containing alkoxysilane, amino group-containing alkoxysilane and epoxy group-containing alkoxysilane is dissolved in water, and JP-A 2001-181599 discloses a modifying agent based on an organosilane compound.

Further examples of coating solutions and their applications can be found in the following documents.

EP 0 535 649 Al describes an air bag coating composition that is provided in the form of an aqueous silicone emulsion composition comprising (A) an aqueous emulsion of a hydroxyl-containing organopolysiloxane, (B) a reaction product of an amino-functional silane or a hydrolyzate thereof with an acid anhydride, an epoxy-functional silane or a hydrolyzate thereof, or an organosilane having an isocyanate radical and a hydrolyzable radical in a molecule or a hydrolyzate thereof, (C) colloidal silica, and (D) a curing catalyst. The composition is applied and cured to an air bag base fabric to form a tack-free, crack resistant coating thereon.

US 4,517,375 (A) describes solutions of silanols prepared by hydrolysis of alkyltrialkoxysilanes whose alkoxy groups are partially or completely hydrolyzed. Preferred hydrolyzed alkyltrialkoxysilanes are those of the formula $CH_3\text{-}CH_2\text{-}CH_2\text{-}Si(OH)_n(OH')_{3-n}$.

US 4,170,690 (A) describes a weatherable coating composition for imparting abrasion resistance to thermoplastic substrates. It comprises about 30-50 parts by weight of a colloidal silica and about 50 to 70 parts by weight of a mixture of (i) dialkyldialkoxysilane and (ii) alkyl trialkoxysilane wherein the weight ratio of (i) to (ii) is about 1:19 to about 1:4.

GB 0 781 488 (A) describes a composition having reduced structure and lower knit time comprising an organopolysiloxane convertible to the cured, solid, elastic state, a structure inducing filler, and 0.01 to 10 per cent by weight of the siloxane of either a silanol of the formula $RnSi(OH)_{4-n}$ or a siloxanol of formula $HO\text{-}Si(R)_2O\text{-}(Si(R)_2O)_m\text{-}H$.

EP 1 179 633 A describes a method for the manufacture of a coated material having appropriate strength, a good light transmitting property and a good water repelling property. This can be achieved by applying to a fiber material a silane-type coating solution and hardening/solidifing it by action of a catalyst.

Summary of the Invention

The present invention relates to a fiber-treating agent. The first aspect of the invention relates to a fiber-treating agent containing an alkoxysilane (a), an organic acid (b) and water (c), wherein 50% or more by weight of the component (a) is an alkoxysilane represented by the following formula (1) (referred to hereinafter as alkoxysilane (1) ) :

$$R^1{}_pSi(OR^2)_{4-p} \qquad (1)$$

wherein $R^1$ represents a C1 to C6 linear or branched alkyl group, a phenyl group, or a C2 to C6 linear or branched alkenyl group, $R^2$ represents a C1 to C6 linear or branched alkyl group, $R^1$s whose number is p may be the same as or different from one another, $R^2$s whose number is (4-p) may be the same as or different from one another, and p is an integer of 1 to 3, and the number of moles of the component (c) is 3 times or more as large as that of the component (a) and wherein the amount of component (c) is 30 to 99.9% by weight of the fiber-treating agent.

The second aspect of the invention relates to the fiber-treating agent above having a pH value of 2 to 5 at 20˚C.

The present invention also provides a method of treating fibers with the fiber-treating agent of the second aspect of the invention and then fibers treated by this method.

Further, the present invention provides use of the fiber-treating agent of the second aspect of the invention for use as a fiber-treating agent.

Detailed description of the invention

For pursuing hydrophobation in the method described in JP-A 2003-342875 supra, water absorption is reduced, the original feeling of fibers is worsened, and comfort at the time of wearing and use is deteriorated. In addition, the Treatment of fibers with an alkali metal hydroxide is necessary, and thus the fibers are denatured and damaged, and the original state of the fibers is hardly maintained.

There is a description that when the composition in JP-A 2005-89882 is applied to fibers, the feeling of the fibers at the time of wearing is improved, but the silicone-based copolymer adheres to the surfaces of the fibers, thus conferring water repellency on the fibers, and therefore, the composition has still not arrive at performance of satisfying both water absorption and quick-drying properties.

The method disclosed in JP-A 2002-61094 supra is a method of coating the surfaces of fibers with a silicon compound, and the resulting fibers are fixed with the silicon compound and do not maintain the original feeling or softness of the fibers.

The methods disclosed in JP-A 9-249748 and JP-A 2001-181599 supra also include coating the surfaces of fibers with a silicon compound having a water-repellant functional group, thus failing to attain natural feeling.

As described above, there is no known method of treating fibers while maintaining the original state of the fibers.

The object of the present invention is to provide a novel fiber-treating agent which can confer quick-drying properties, softness and/or toughness on fibers while maintaining the original water absorption of the fibers.

The present inventors found that a fiber-treating agent containing a specific alkoxysilane, an organic acid and water can suitably regulate the polymerization rate of a silanol compound formed by hydrolysis of the alkoxysilane, and as a result, the silanol compound is allowed to penetrate into the fibers and polymerized in the inside of the fibers, whereby a polymer of the silanol compound can be contained in the inside of the fibers without filling the polymer of the silanol compound in gaps among the fibers.

According to the present invention, fibers can be endowed with quick-drying properties, softness and/or toughness, while the original state of the fibers is maintained.

The present invention relates to a method of treating fibers with the fiber-treating agent in the first aspect of the invention, and use thereof as a fiber-treating agent.

The present invention provides a method of treating fibers, including step (i) of bringing the fiber-treating agent in the second aspect of the invention into contact with fibers to penetrate, into the fibers, a silanol compound represented by the following formula (4) (referred to hereinafter as silanol compound (4)):

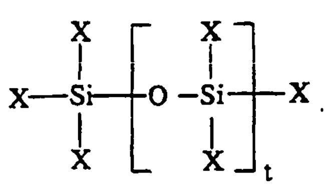

(4)

wherein X is a group represented by $R^1$, $OR^2$ or OH, t is an integer of 0 to 2, X's whose number is (2t + 4) may be the same as or different from one another, and at least one of X's is OH, and $R^1$ and $R^2$ have the same meanings as defined above, and step (ii) of polymerizing the silanol compound (4), as well as fibers treated by this method.

[Component (a)]

In the alkoxysilane as the component (a) in the present invention, 50 % or more by weight of the alkoxysilane is the alkoxysilane (1), preferably 60 % or more by weight of the alkoxysilane is the alkoxysilane (1), more preferably 80 % by weight or more of the alkoxysilane is the alkoxysilane (1), even more preferably 100 % by weight of the alkoxysilane is the alkoxysilane (1).

In the alkoxysilane (1), the alkyl group represented by $R^1$ or $R^2$ includes a methyl group, ethyl group, propyl group, butyl group, isopropyl group, isobutyl group, t-butyl group etc., the alkenyl group represented by $R^1$ includes a vinyl group, allyl group etc., and a phenyl group can also be mentioned as $R^1$. From the viewpoint of penetration into the inside of the fiber, $R^1$ is preferably a C1 to C6 alkyl group, more preferably a C1 or C2 alkyl group. From the viewpoint of safety of byproducts generated by hydrolysis, reactivity of hydrolysis reaction, etc. , $R^2$ is preferably a C1 to C4 alkyl group, more preferably a C1 to C2 alkyl group. p is preferably 1 to 2.

As the component (a), the trialkoxysilane (a1) represented by the formula (2) and the dialkoxysilane (a2) represented by the formula (3) may be used alone, but from the viewpoint of not only conferring quick-drying properties on fibers but also maintaining water absorption to improve feeling at use, both the trialkoxysilane (a1) and dialkoxysilane (a2) are preferably contained.

$$R^1Si(OR^2)_3 \qquad (2)$$

$$R^1_2Si(OR^2)_2 \qquad (3)$$

wherein $R^1$ and $R^2$ have the same meanings as defined above.

The trialkoxysilane (a1) is preferably alkyl (C1 to C6) trimethoxysilane or alkyl (C1 to C6) triethoxysilane, more preferably methyl trimethoxysilane, ethyl triethoxysilane or propyl triethoxysilane. The dialkoxysilane (a2) is preferably dialkyl (C1 to C6) dimethoxysilane, dialkyl (C1 to C6) diethoxysilane or the like, more preferably dimethyl dimethoxysilane or diethyl diethoxysilane.

The trialkoxysilane (a1)/dialkoxysilane (a2) ratio by weight is preferably 9/1 to 1/9, more preferably 9/1 to 3/7, still more preferably 8/2 to 4/6, further more preferably 7/3 to 5/5.

[Component (b)]

Examples of the organic acid as the component (b) in the present invention include oxalic acid (pKa = 1.04, 3.82), maleic acid (pKa = 1.75, 5.83), tartaric acid (pKa = 2.82, 3.96), fumaric acid (pKa 2.85, 4.10), citric acid (pKa = 2.90, 4.34), malic acid (pKa = 3.24, 4.71), succinic acid (pKa 4.00, 5.24), formic acid (pKa = 3.55), lactic acid (pKa = 3.66), adipic acid (pKa = 4.26, 5.03), acetic acid (pKa = 4.56) and propionic acid_ (pKa = 4.67), and particularly the organic acid as the component (b) is preferably an organic acid having a first dissociation (pKa1) in the range of 2.9 to 5.0, more preferably an organic acid having a pKa1 in the range of 3.5 to 5.0. Among these organic acids, adipic acid, malic acid, acetic acid and propionic acid whose hydrolysis reaction and polymerization reaction with the alkoxysilane (1) can be easily regulated are preferable, and adipic acid with less smell is particularly preferable.

[Fiber-treating agent]

The fiber-treating agent of the present invention is obtained by mixing the alkoxysilane (a), the organic acid (b) and water (c). The fiber-treating agent of the present invention contains, before hydrolysis, the alkoxysilane (a), the organic acid (b).and water (c), and after hydrolysis, contains the silanol compound (4) formed by hydrolysis of the alkoxysilane (1), the organic acid (b) and water (c). When the fiber-treating agent of the present invention is a 2-agent system, the fiber-treating agent is composed of a first agent containing the alkoxysilane (a) and a second agent containing the organic acid (b) and water (c).

The amount of the alkoxysilane (a) in the fiber-treating agent of the present invention is preferably 0.1 % or more by weight, more preferably 2 % or more by weight, or is preferably 82 % or less by weight, more preferably 58 % or less by weight, based on the amount of the fiber-treating agent of the present invention (in the case of the 2-agent system, "the amount of the fiber-treating agent" refers to the total amount of the first and second agents; this hereinafter applies). The content of the alkoxysilane (a) in the first agent is preferably 70 to 100 % by weight, more preferably 80 to 100 % by weight still more preferably 90 to 100 % by weight, from the viewpoint of storage stability.

From the viewpoint of suppression of the polymerization reaction, the amount of the organic acid (b) in the fiber-treating agent of the present invention is preferably 0.001 to 5 % by weight, more preferably 0.001 to 1 % by weight. When the fiber-treating agent of the present invention is a 2-agent system, the organic acid (b) is preferably incorporated not into the first agent but into the second agent only, from the viewpoint of solubility and storage stability.

The amount of water (c) in the fiber-treating agent of the present invention is 30 % or more by weight, more preferably 50 % or more by weight, still more preferably 70 % or more by weight, from the viewpoint of sufficiently swelling the fibers and sufficiently penetrating, into the fibers, the silanol compound (4) formed by hydrolysis of the alkoxysilane (1). The upper limit is 99.9 % or less by weight, more preferably 95 % or less by weight, still more preferably 85 % or less by weight.

The molar ratio of water (c) to the alkoxysilane (a) is preferably 3 or more, preferably 10 to 1000, more preferably 25 to 600, from the viewpoint of sufficiently penetrating, into the fibers, the silanol compound (4) formed by hydrolysis of the alkoxysilane (1).

When the fiber-treating agent of the present invention is a 2-agent system, the water in the fiber-treating agent of the present invention is preferably incorporated not into the first agent but into the second agent only.

The fiber-treating agent of the present invention preferably contains a surfactant (d) for improving the dispersion of the alkoxysilane (a) into an aqueous phase and for promoting the hydrolysis reaction. As the surfactant, it is possible to use a nonionic surfactant, an anionic surfactant, a cationic surfactant and/or an amphoteric surfactant.

The nonionic surfactant includes polyoxyalkylene alkyl ether, polyoxyalkylene alkenyl ether, higher fatty acid sucrose ester, polyglycerin fatty acid ester, higher fatty acid mono- or diethanol amide, polyoxyethylene hardened castor oil, polyoxyethylene sorbitan fatty acid ester, polyoxyethylene sorbitol fatty acid ester, alkyl saccharide-based surfactants, alkylamine oxide, alkylamide amine oxide etc. Among these; polyoxyalkylene alkyl ether and polyoxyethylene hardened castor oil are preferable, and polyoxyethylene alkyl ether is particularly preferable.

The anionic surfactant includes alkyl benzene sulfonate, alkyl or alkenyl ether sulfate; alkyl or alkenyl sulfonate, olefin sulfonate, alkane sulfonate, saturated or unsaturated fatty acid salt, alkyl or alkenyl ether carboxylic acid salt, α-sulfone fatty acid salt, N-acylamino acid-based surfactants , phosphoric acid mono- or diester-based surfactants, sulfosuccinic acid ester, etc. The counterion of the anionic surfactant includes an alkali metal ion such as sodium ion, potassium ion etc. ; an alkaline earth metal ion such as calcium ion, magnesium ion etc. ; ammonium ion; an alkanol amine having one to three C2 or C3 alkanol groups (for example, monoethanolamine, diethanolamine, triethanolamine, triisopropanolamine etc.).

The cationic surfactant includes quaternary ammonium salts represented by the following formula (5):

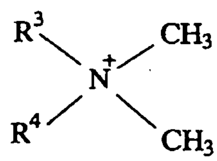 An$^-$ (5)

wherein $R^3$ and $R^4$ independently represent a hydrogen atom, a C1 to C28 alkyl group or a benzyl group provided that both $R^3$ and $R^4$ are not simultaneously hydrogen atoms, benzyl groups or C1 to C3 lower alkyl groups, and An- represents an anion.

In the formula (5), one of $R^3$ and $R^4$ is preferably a C16 to C24 (especially C22) alkyl group, particularly preferably a linear alkyl group, and the other is preferably a C1 to C3 lower alkyl group, particularly preferably a methyl group. The anion An$^-$ represents a halide ion such as chloride, ion, bromide ion and iodide ion, and an organic anion such as methyl sulfate ion, ethyl sulfate ion, methyl carbonate ion and saccharinate ion, preferably a halide ion, particularly preferably a chloride ion.

The cationic surfactant is preferably a long monoalkyl quaternary ammonium salt, and specific examples include cetyltrimethyl ammonium chloride, stearyltrimethyl ammonium chloride, aralkyltrimethyl ammonium chloride, behenyltrimethyl ammonium chloride etc., among which stearyltrimethyl ammonium chloride and behenyltrimethyl ammonium chloride are preferable.

The amphoteric surfactant includes surfactants based on imidazoline, carbobetaine, amidobetaine, sulfobetaine, hydroxysulfobetaine, amidosulfobetaine, etc.

The surfactant (d) is preferably a nonionic surfactant having an HLB value of 9 to 15, particularly 11 to 14, from the emulsification ability (miscibility of the alkoxysilane (a), the organic acid (b), water (c) and the surfactant (d)). The HLB is a value calculated according to the Griffin method.

Two or more surfactants can be used in combination, and the content of the surfactant(s) in the fiber-treating agent of the present invention is preferably 0.1 to 20 % by weight, more preferably 0.5 to 15 % by weight, still more preferably 1 to 10 % by weight, from the viewpoint of emulsification for mixing and promotion of hydrolysis.

For the purpose of dissolving the silanol compound (4) formed by hydrolysis of the alkoxysilane, (1), the fiber-treating agent of the present invention can also contain a water-soluble organic solvent such as a C1 to C3 lower

monovalent alcohol such as methanol, ethanol etc. and a C2 to C4 polyvalent alcohol such as glycerin etc. The content of the water-soluble organic solvent in the fiber-treating agent of the present invention is preferably 35 % or less by weight, more preferably 20 % or less by weight, from the viewpoint of sufficiently swelling the fibers and sufficiently penetrating the silanol compound (4) into the fibers. In addition, the fiber-treating agent after hydrolysis of the alkoxysilane (1) contains $R^2OH$ ($R^2$ has the same meaning as defined above) in a byproduct.

The fiber-treating agent of the present invention can be compounded suitably with a pH adjusting agent, a lubricant, a silicone derivative, a cationic polymer, a humectant, a viscosity-regulating agent, a perfume, a coloring agent, an UV light absorber, an antioxidant, an antibacterial agent etc. , depending on the object.

In the fiber-treating agent of the present invention, there are necessity for hydrolysis of the alkoxysilane (1) to form the silanol compound (4) and necessity for retarding the polymerization reaction in order to penetrate the silanol compound (4) into the fibers to polymerize it in the fibers. Accordingly, the pH value at 20˚C is regulated in the range of 2 to 5, preferably 2 to 4. In the case of the 2-agent system, the pH of the second agent at 20˚C is regulated in the above range.

From the viewpoint of securing the stability of the fiber-treating agent for a long period, the form of the fiber-treating agent of the present invention is preferably a 2-agent system containing a first agent containing the alkoxysilane (a) wherein 50% or more by weight of the component, (a) is the alkoxysilane (1) and a second agent having a pH value of 2 to 5 at 20˚C containing the organic acid (b) and water (c). In the present invention, the "2-agent system" refers to the form in which the alkoxysilane (a) in the first agent is separated from the organic acid (b) and water (c) in the second agent. In both the first and second agents, the individual components (for example, the trialkoxysilane (a1) and dialkoxysilane (a2) described later) may be provided in such a state that they are separated from each other.

Just before use, the fiber-treating agent maybe prepared by mixing the alkoxysilane (a), the organic acid (b), water (c) and if necessary the surfactant (d) or other arbitrary components, followed by adjusting the resulting mixture to pH 2 to 5.

When the fiber-treating agent of the present invention is formed into a 2-agent system, the surfactant (d) is contained preferably in the second agent, but when the first agent does not contain water, the surfactant (d) may be contained in the first agent. Other arbitrary components are contained , preferably in the second agent, but non-aqueous liquid components or solid components may be incorporated into the first agent.

The fiber-treating agent of the present invention is useful as an agent conferring quick-drying properties on fibers, a softness conferring agent and/or a toughness conferring agent. The "toughness" can be evaluated in terms of wear resistance or prevention of removal of down.

[Method of Producing the Fiber-Treating Agent]

When the fiber-treating agent of the present invention is prepared just before use by mixing the alkoxysilane (a), the organic acid (b), water (c) and if necessary the surfactant (d) or other arbitrary components, the order of mixing them is not particularly limited, but it is preferable that the organic acid (b), water (c) and if necessary the surfactant (d) are mixed and then the alkoxysilane (a) is mixed in order that the polymerization of the silanol compound (4) formed by hydrolysis of the alkoxysilane (1) is retarded and the penetration thereof into the inside of the fiber proceeds sufficiently.

When both the trialkoxysilane (a1) and dialkoxysilane (a2) are used as the alkoxysilane (a), it is preferable that the trialkoxysilane (a1), the organic acid (b) and water (c) are first mixed and then the dialkoxysilane (a2) is mixed. It is preferable that after the trialkoxysilane (a1), the organic acid (b) and water (c) are mixed and before the dialkoxysilane (a2) is mixed, at least part of the trialkoxysilane (a1) is hydrolyzed. Progress of the hydrolysis can be judged in terms of an increase in the transparence of the liquid.

By mixing the alkoxysilane (a), the organic acid (b), water (c) and, if necessary, the surfactant (d) or other arbitrary components, the alkoxysilane (1) is converted by hydrolysis into the silanol compound (4) capable of penetrating into the fibers.

[Fiber-Treating Method and Treated Fibers]

The method of treating fibers according to the present invention includes step (i) of bringing the fiber-treating agent according to the present invention into contact with fibers to penetrate, into the fibers, the silanol compound (4) formed by hydrolysis of the alkoxysilane (1) and step (ii) of polymerizing the silanol compound (4).

In the silanol compound (4), it is preferable from the viewpoint of its physical properties and penetration into the fibers that at least one of X's whose number is (2t + 4) is OH, and (t + 2) or more X's are OH groups. t is an integer of 0 to 2, and from the viewpoint of penetration into the fibers, t is preferably 0.

For easy penetration into the fibers, the molecular weight of the silanol compound (4) is preferably 300 or less, more preferably 200 or less, and is preferably 90 or more.

After hydrolysis, the content of the silanol compound (4) in the fiber-treating agent of the present invention is

preferably 0.1 % or more by weight, more preferably 2 % or more by weight and is preferably 69 % or less by weight, more preferably 49 % or less by weight.

Fibers which can be suitably treated in the present invention include vegetable fibers such as cotton, hemp etc.; animal fibers such as wool, silk etc.; regenerated fibers or semi-synthetic fibers such as rayon, acetate etc.; and paper-making fibers such as pulp, camellia, SANA, kenap, cotton linter etc.

To bring the fiber-treating agent of the present invention into contact with fibers in step (i), it is preferable, that the alkoxysilane (a), the organic acid (b), water (c) and if necessary the surfactant (d) or other arbitrary component are mixed before use and then stirred under shaking by means such as a shaker, and after it is confirmed that the mixed solution becomes transparent by observation with the naked eye, the resulting mixture is contacted with fibers.

In the case of the 2-agent system, the mixing ratio of the first agent to the second agent (the first agent/second agent ratio by weight) is preferably 80/20 to 1/99, more preferably 60/40 to 20/80. The mixture is in a turbid emulsified or partially emulsified state just after mixing and is then left or stirred if necessary to turn transparent by which the hydrolysis of the alkoxysilane (1) and formation of the silanol compound (4) can be confirmed;

When the resulting mixture is left, the polymerization reaction of the silanol compound (4) will proceed, and thus the resulting mixture is contacted with fibers within 24 hours, preferably within 12 hours, more preferably within 3 hours, further more preferably within 1 hour. The silanol compound (4) can thereby be penetrated into the fibers . The method of contacting the treating agent with fibers includes a method of dipping fibers in the treating agent, a method of spraying fibers with the treating agent, and a method of coating fibers with the treating agent. The fibers to be contacted with the treating agent may be wet or dry.

The silanol compound (4) upon contacting with fibers for several seconds penetrates sufficiently into the fibers and may be left for 1 minute to 2 hours for more uniform penetration.

In step (ii), the silanol compound (4) is allowed to penetrate sufficiently into the fibers; and then the silanol compound (4) in the inside of the fiber is polymerized.

The polymerization can be promoted by heating, and is conducted by heating preferably at 60˚C or more, more preferably at 80 to 200˚C. As the temperature is increased, the polymerization proceeds in a shorter time. Specifically, hot-air drying and press heating can be mentioned.

Alternatively, the polymerization can be promoted by adjusting the pH of the treating agent to 0-2 or 5-12. 5 without drying the fibers. While the fibers are dipped in the treating agent or after an excess of the liquid is removed, the pH can be regulated with an acid or a base.

After the polymerization step, the fibers are further washed with water to remove an excess of the polymerized product and can maintain their higher original feeling.

Preferably the method further has step (iii) of washing the fibers between steps (i) and (ii). By further conducing step (iii), an excess of the silanol compound (4) on the surfaces of fibers can be removed, and the original feeling of the fibers can be maintained.

The fibers treated by the method of treating fibers according to the present invention can contain a polymer of the silanol compound (4) in a larger amount in the inside of the fiber than in the surface of the fiber. The distribution of the polymer of the silanol compound (4) can be measured according to an energy dispersive X-ray spectroscope (EDS) described later. According to the fiber-treating method of the present invention, the inside of the fiber can be modified and the treated fibers are excellent in quick-drying properties, softness, toughness etc.

## Brief Description of the drawings

Fig. 1 shows $^{29}$Si NMR spectrums just after preparation and 0 to 1 hour, 4 to 5 hours and 1 day after preparation of the treating solution obtained in Example 1.

Fig. 2 is an SEM photograph of the treated cloth obtained in Example 1 (cotton broad cloth, treating agent C1, methyltriethoxysilane/dimethyldiethoxysilane = 10/1).

Fig. 3 shows a silicon mapping photopicture of the treated cloth obtained in Example 1 (cotton broad cloth, treating agent C1, methyltriethoxysilane/dimethyldiethoxysilane = 10/0).

Fig. 4 shows an enlarged silicon mapping photopicture of the treated cloth obtained in Example 1 (cotton broad cloth, treating agent C1, methyltriethoxysilane/dimethyldiethoxysilane = 10/0).

Fig. 5 is an SEM photopicture of the treated cloth obtained in Example 2 (cotton broad cloth, treating agent C2, methyltriethoxysilane/dimethyldiethoxysilane = 7/3).

Fig. 6 shows a silicon mapping photopicture of the treated cloth obtained in Example 2 (cotton broad cloth, treating agent C2, methyltriethoxysilane/dimethyldiethoxysilane = 7/3).

Fig. 7 is an SEM photopicture of the treated cloth, obtained in Example 3 (wool, treating agent C3; methyltriethoxysilane/dimethyldiethoxysilane = 10/0).

Fig. 8 shows a silicon mapping photopicture of the treated cloth obtained in Example 3 (wool, treating agent C3, methyltriethoxysilane/dimethyldiethoxysilane = 10/0).
Fig. 9 is an SEM photopicture of the treated cloth obtained in Example 4 (wool, treating agent C4, methyltriethoxysilane/dimethyldiethoxysilane = 7/3).
Fig. 10 shows a silicon mapping photopicture of the treated cloth obtained in Example 4 (wool, treating agent C4, methyltriethoxysilane/dimethyldiethoxysilane = 7/3).
Fig. 11 is an SEM photopicture of the treated cloth obtained in Comparative Example 1 (cotton broad cloth, coating agent CC1).
Fig. 12 shows a silicon mapping photopicture of the treated cloth obtained in Comparative Example 1 (cotton broad cloth, coating agent CC1).
Fig. 13 shows an enlarged silicon mapping photopicture of the treated cloth obtained in Comparative Example 1 (cotton broad cloth, coating agent CC1).
Fig. 14 is an SEM photopicture of the treated paper obtained in Example 5 (paper, treating agent C5, methyltriethoxysilane/dimethyldiethoxysilane = 10/0).
Fig. 15 shows a silicon mapping photopicture of the treated paper obtained in Example 5 (paper, treating agent C5, methyltriethoxysilane/dimethyldiethoxysilane = 10/0).
Fig. 16 shows an enlarged silicon mapping photopicture of the treated paper obtained in Example 5 (paper, treating agent C5, methyltriethoxysilane/dimethyldiethoxysilane = 10/0).
Fig. 17 is an SEM photopicture of the treated paper obtained in Comparative Example 2 (paper, treating agent CC2, methyltriethoxysilane/dimethyldiethoxysilane = 10/0).
Fig. 18 shows a silicon mapping photopicture of the treated paper obtained in Comparative Example 2 (paper, treating agent CC2, methyltriethoxysilane/dimethyldiethoxysilane = 10/0).
Fig. 19 shows an enlarged silicon mapping photopicture of the treated paper obtained in Comparative Example 2 (paper, treating agent CC2, methyltriethoxysilane/dimethyldiethoxysilane = 10/0).

## Examples

The present invention is described by reference to the Examples below. The Examples are mere illustrative of the present invention and not intended'to limit the present invention.

In the Examples, "%" is % by weight unless otherwise specified. ,

## Preparation Example of Catalyst Solution

Adipic acid; water and if necessary polyoxyethylene lauryl ether (Emulgen 108 manufactured by Kao Corporation) were mixed with one another to prepare catalyst solutions B1 to B4 (second agent) having the compositions shown in Table 1.

Table 1

| | | Adipic acid | Polyoxyethylene lauryl ether (Emulgen 108) | Water | pH |
|---|---|---|---|---|---|
| Catalyst solutions | B1 | 1. 0% | 6. 67% | Balance | 2. 75 |
| | B2 | 1. 0% | — | Balance | 2. 55 |
| | B3 | 0. 1% | 6. 67% | Balance | 3. 40 |
| | B4 | 0. 1% | — | Balance | 3. 06 |

Example 1

(1) Synthesis of Treating Solution C1

1.37 g methyltriethoxysilane (LS-1890, manufactured by Shin-Etsu Chemical Co., Ltd.; hereinafter, methyltrimethoxysilane refers to this commercial product) was added to 4.11 g catalyst solution B and stirred for 10 minutes until the turbid suspension turned transparent, to give a treating solution C1. The composition of the resulting treating solution (which refers to the composition before hydrolysis; hereinafter this applies) is shown in Table 2.

For confirming the formation of silanol in the resulting treating solution, its $^{29}$Si NMR (UNITY INOVA 300, manufactured by Varian) spectrums were measured just after preparation and 0 to 1 hour, 4 to 5 hours, and 1 day after preparation. The results are shown in Fig. 1.

In the $^{29}$Si NMR spectrum, a peak of Si in the trihydroxyalkyl silane appears in the vicinity of 37 ppm, a peak of Si in the dihydroxyalkylsiloxy group in the vicinity of 46 ppm, and a peak of Si in the monohydroxyalkylsiloxy group in the vicinity of 56 ppm. From the amount of Si in the trihydroxyalkyl silane, the presence of the alkyl silanol monomer can be confirmed.

0 to 1 hour after preparation, the distribution was 64% monomer, 33% dimer, and 3% trimer or more. 4 to 5 hours after preparation, the distribution was 48% monomer, 43% dimer, and 7% trimer or more. 1 day after preparation, the distribution was 28% monomer, 41% dimer, and 31% trimer or more. In the following fiber treatment, the treating solution just after preparation was used.

(2) Treatment of Cotton Broad Cloth

5.48 g of the treating solution C was applied onto 5.48 g cotton broad cloth pretreated by a method shown below, and then left at room temperature for 60 minutes and dried at 80˚C for 2 hours. The amount of the alkoxysilane based on the cloth was 25 % by weight. The dried cloth was washed with a clothing detergent (Liquid Attack, manufactured by Kao Corporation) (washing conditions: 30 g detergent, 45 L tap water was used, washing for 5 minutes → rinsing once with running water → dehydration for 3 minutes) and then air-dried in a room to give a treated cloth. The increase in the weight of the cloth after treatment was 5.5%.

<Method of Pretreatment of Cotton Broad Cloth>

A cotton broad cloth (manufactured by Yato Shoten) was washed 10 times with a commercial.detergent ("Attack" manufactured by Kao Corporation) in a two-bath washing machine (VH-360S1 manufactured by Toshiba Corporation) (detergent concentration of 0.0667 % by weight, 40 L tap water, water temperature of 20˚C, washing for 10 minutes → rinsing with running water for 15 minutes → dehydration for 5 minutes) and then air-dried. This cloth was cut into a piece of 15 cm x 25 cm to give a pretreated cloth.

(3) Observation under SEM

The treated cloth was embedded in epoxy resin, then hardened and cut into a section with a microtome (ULTRACUTTER manufactured by LEICA) and made electrically conductive by deposition with Pt-Pd. This fiber section was observed under a field emission scanning electron microscope (FE-SEM: S4800, manufactured by Hitachi, Ltd., accelerating voltage of 15 kV, probe current High, focus mode HR, condenser lens 5, aperture 1). The results are shown in Fig. 2. The surface analysis of silicon was carried out with an energy dispersive X-ray spectroscope (EDS) (EMAX ENERGY EX-350 manufactured by Horiba, mapping measurement time 1500 seconds, process time 5). The results are shown in Fig. 3, and its enlarged photopicture is shown in Fig. 4.

In the SEM photopicture in Fig. 2, a section of the cotton fibers is observed. In the Si mapping photopicture in Fig. 3, it was observed that silicon is distributed mainly in the inside of the cotton fiber and occurs scarcely in gaps among the fibers. In the enlarged Si mapping photopicture in Fig. 4, a profile of silicon concentration is shown, and it was observed that the silicon concentration is higher in the inside of the fiber, and silicon occurs scarcely in gaps among the fibers.

Example 2

(1) Synthesis of Treating Solution C2

1.00 g methyltriethoxysilane was added to 4.28 g catalyst solution B and stirred for 20 minutes until the turbid suspension turned colorless and transparent. 0.43 g dimethyldiethoxysilane (LS-1370 manufactured by Shin-Etsu Chemical Co., Ltd.; hereinafter, dimethyldiethoxysilane refers to this commercial product) was added thereto and stirred for 3 minutes until the suspension turned transparent again, to give a treating solution C2. The composition of the resulting treating solution is shown in Table 2.

(2) Treatment of Cotton Broad Cloth

5.71 g cotton broad cloth pretreated in the same manner as in Example 1 was dipped in 5.71 g of the treating solution C2, then left at room temperature for 60 minutes and dried at 80˚C for 2 hours. The amount of the alkoxysilane based on the cloth was 25 % by weight. The dried cloth was washed with a clothing detergent (Liquid Attack, manufactured by Kao Corporation) and then air-dried in a room to give a treated cloth. The increase in the weight of the cloth after treatment was 2.1%.

(3) Observation under SEM

The treated cloth was embedded in epoxy resin and observed for its fiber section under a scanning microscope in the same manner as in Example 1. In the SEM photopicture in Fig. 5 and the Si mapping photopicture in Fig. 6, it was observed that silicon is distributed mainly in the inside of the cotton fiber and occurs scarcely in gaps among the fibers.

Example 3

(1) Synthesis of Treating Solution C3

3.11 g methyltriethoxysilane was added to 9.33 g catalyst solution B and stirred for 10 minutes until the turbid suspension turned transparent, to give a treating solution C3. The composition of the resulting treating solution is shown in Table 2.

(2) Treatment of wool Jersey

12.44 g of the treating solution C3 was applied onto 12.44 g wool jersey (wool jersey knit cloth (manufactured by Yato Shoten) cut in a size of 2.0 cm × 2.0 cm), then dried at room temperature for 60 minutes and dried at 80˚C for 2 hours. The amount of the alkoxysilane based on the cloth was 25 % by weight. The dried cloth was washed with a clothing detergent (Liquid Attack, manufactured by Kao Corporation) and then air-dried in a room to give a treated cloth. The increase in the weight of the cloth after treatment was 8.4%.

(3) Observation under SEM

The treated cloth was embedded in epoxy resin and observed for its fiber section under a scanning microscope in the same manner as in Example 1. In the SEM photopicture in Fig. 7 and the Si mapping photopicture in Fig. 8, it was observed that silicon is distributed mainly in the inside of the wood fiber and occurs scarcely in gaps among the fibers.

Example 4

(1) Synthesis of Treating Solution C4

1.89 g methyltriethoxysilane was added to 8.09 g catalyst solution B and stirred for 20 minutes until the turbid suspension turned colorless and transparent. 0.81 g dimethyldiethoxysilane was added thereto and stirred for 3 minutes until the suspension turned transparent again, to give a treating solution C4. The composition of the resulting treating solution is shown in Table 2.

(2) Treatment of Wool Jersey

10.79 g of the treating solution C4 was applied onto 10.79 g the same wool jersey as in Example 3, then dried at room temperature for 60 minutes and dried at 80˚C for 2 hours. The amount of the alkoxysilane based on the cloth was 25 % by weight. The dried cloth was washed with a clothing detergent (Liquid Attack, manufactured by Kao Corporation) and then air-dried in a room to give a treated cloth. The increase in the weight of the cloth after treatment was 11.7%.

(3) Observation under SEM

The treated cloth was embedded in epoxy resin and observed for its fiber section under a scanning microscope in the same manner as in Example 1. In the SEM photopicture in Fig. 9 and the Si mapping photopicture in Fig. 10, it was observed that silicon is distributed mainly in the inside of the wood fiber and occurs scarcely in gaps among the fibers.

Comparative Example 1

(1) Synthesis of Coating Solution CC1

181 g methyltrimethoxysilane, 50 g methanol and 18 g water were added and stirred. While 2 g of 61% nitric acid was added thereto, the mixture was stirred at 80˚C for 3 hours. Thereafter, the container was depressurized thereby removing the methanol, to produce a methyltrimethoxysilane oligomer. Regarding the degree of condensation, this oligomer was estimated to be a trimer or a tetramer.

0.8 g dibutyltin acetate and 20 g isopropyl alcohol were added to, and sufficiently mixed with, 19 g of the resulting methyltrimethoxysilane oligomer, to prepare a coasting solution CCl.

(2) Treatment of Cotton Broad Cloth

3.0 g of the coating solution CC1 was applied onto 5.7 g cotton broad cloth pretreated in the same manner as in Example 1, and then left at room temperature for 10 minutes and dried at 130˚C for 2 hours. The dried cloth was washed with a clothing detergent (Liquid Attack, manufactured by Kao Corporation) and then air-dried in a room to give a treated cloth. The increase in the weight of the cloth after treatment was 11.3%.

(3) Observation under SEM

The treated cloth was embedded in epoxy resin and observed for its fiber section under a scanning electron microscope in the same manner as in Example 1. In the SEM photopicture in Fig. 11, a section of the cotton fibers is observed. A filling considered as polysiloxane is observed among the fibers, and as can be seen from the Si mapping photopicture in Fig. 12, silicon is present between fibers to bind the fibers in the form of a binder. On the other hand, silicon is not observed in the inside of the cotton fiber, to show that polysiloxane does not penetrate into the cotton fiber. The enlarged Si mapping photopicture in Fig. 13 shows a profile of silicon concentration, and it is observed that silicon occurs scarcely inside of the fiber and occurs at higher concentration in gaps among the fibers.

The part observed in the center of the fiber in the enlarged photopicture is a hollow part called lumen, and this part is not regarded as the inside of the fiber.

Example 5

(1) Synthesis of Treating Solution C5

10 g methyltriethoxysilane was added to 1.20 g catalyst solution B and stirred for 10 minutes until the turbid suspension turned transparent, to give a treating solution C5. The composition of the resulting treating solution is shown in Table 2.

(2) Treatment of Paper

5.3 g paper obtained by a method described below was dipped in 30 g of the treating solution C5, raised after 30 seconds, air-dried at room temperature for 10 minutes and dried at 80˚C for 2 hours. The increase in the weight of the paper after treatment was 38.7%.

<Method of Producing the Paper>

Laubholz bleached kraft pulp (abbreviated hereinafter as LBKP) was dissociated and beaten at room temperature to give 2.2% LBKP slurry. The Canadian standard freeness of the slurry was 420 ml. 2.2% LBKP slurry was weighed out such that the basis weight of a sheet after paper making became 85 g/$m^2$ on an oven-dry weight basis. The slurry was diluted to a pulp density of 0.5% with water and used to produce paper with a 150-mesh wire in a rectangular TAPPI paper making machine, followed by coating to give wet paper. The wet paper after paper making was pressed at 3.5 kg/$cm^2$ for 5 minutes with a pressing machine and then dried at 105˚C for 2 minutes with a drum

dryer. The water content of the dried paper was regulated for 1 day under the conditions of 23˚C and 50% humidity.

(3) Observation under SEM

The treated paper was embedded in epoxy resin and observed for its fiber section under a scanning microscope in the same manner as in Example 1. In the SEM photopicture in Fig. 14 and the Si mapping photopicture in Fig. 15, it was observed that silicon is distributed mainly in the inside of the pulp fiber and occurs scarcely in gaps among the fibers. In the enlarged Si mapping photopicture in Fig. 16, a profile of silicon concentration is shown, and it was observed that the silicon concentration is higher in the inside of the fiber and silicon occurs scarcely in gaps among the fibers.

Comparative Example 2

(1) Synthesis of Coating Solution CC2

9.5 g of the methyltrimethoxysilane oligomer obtained in Comparative Example 1 was added to', and sufficiently mixed with, a mixed solution of 0.4 g dibutyltin acetate and 10 g isopropyl alcohol, to prepare a coating solution CC2.

(2) Treatment of Paper

5.3 g of the same paper as in Example 5 was dipped in 9.9 g of the coating solution CC2, raised after 30 seconds, air-dried at room temperature for 10 minutes and dried at 130˚C for 60 minutes. The increase in the weight of the paper after treatment was 60.4 % by weight.

(3) Observation under SEM

The treated paper was embedded in epoxy resin and observed for its fiber section under a scanning electron microscope in the same manner as in Example 1. As can be seen from the SEM photopicture in Fig. 17 and the Si mapping photopicture in Fig. 18, silicon occurs among the pulp fibers and binds the fibers in the form of a binder. On the other hand, silicon is not observed inside of the pulp fiber, to show that polysiloxane does not penetrate into the pulp fiber. The enlarged Si mapping photopicture in Fig. 19 shows a profile of silicon concentration, and it was observed that silicon occurs scarcely in the inside of the fiber and occurs at higher concentration in gaps among the fibers.

The results in Examples 1 to 5 and Comparative Examples 1 to 2 are collectively shown in Table 2.

Table 2

| | | Example 1 | Example 2 | Example 3 | Example 4 | Example 5 | Comparative example 1 | Comparative example 2 |
|---|---|---|---|---|---|---|---|---|
| Treating solution | | C1 | C2 | C3 | C4 | C5 | Coating solution CC1 3.0g | Coating solution CC2 9.9g |
| Component (a) | methyltriethoxysilane (g) | 1.37 | 1.00 | 3.11 | 1.89 | 10.0 | | |
| | Dimethyldiethoxysilane (g) | - | 0.43 | - | 0.81 | - | | |
| Catalyst solution | Kind | B1 | B3 | B1 | B3 | B1 | | |
| | Amount (g) | 4.11 | 4.28 | 9.33 | 8.09 | 20.0 | | |
| Weight of treating solution (g) | | 5.48 | 5.71 | 12.44 | 10.79 | 30.0 | | |
| Composition | Component (a) (%) | 25.00 | 17.50 | 25.00 | 17.50 | 33.33 | | |
| | Adipic acid (%) | 0.75 | 0.08 | 0.75 | 0.08 | 0.67 | | |
| | Emulgen 108 (%) | 5.00 | 5.00 | 5.00 | 5.00 | 4.44 | | |
| | water (%) | 69.25 | 69.93 | 69.25 | 69.93 | 61.56 | | |
| Methyltriethoxysilane/Dimethyldiethoxysilane (weight ratio) | | 10/0 | 7/3 | 10/0 | 7/3 | 10/0 | | |
| Silicon content (%) | | 3.9 | 4.2 | 3.9 | 4.2 | 5.2 | | |
| Water/component (a) (molar ratio) | | 27.4 | 26.1 | 27.4 | 26.1 | 18.3 | | |
| pH (20˚C) | | 3.0 | 3.6 | 3.0 | 3.6 | 3.2 | | |
| Treated fibers | Kind | Cotton broad | Cotton broad | Wool jersey | Wool jersey | Paper | Cotton broad | Paper |
| | Fiber weight (g) | 5.48 | 5.71 | 12.44 | 10.79 | 5.3 | 5.7 | 5.3 |
| | Treatment amount (%) | 25 | 25 | 25 | 25 | - | - | - |
| | Increase in weight after treatment (%) | 5.5 | 2.1 | 8.4 | 11.7 | 38.7 | 11.3 | 60.4 |

Examples 6 to 11

Treating solutions C6 to C11 having the compositions shown in Table 3 were prepared. The treating solution having methyltriethoxysilane/dimethyldiethoxysilane (10/1) was prepared in the same manner as in Example 1, and the treating solution having methyltriethoxysilane/dimethyldiethoxysilane (7/3) was prepared in the same manner as in Example 2.

Cotton towels pretreated in a method shown below were dipped for 60 minutes in the resulting treating solution and then dried at 80°C for 12 hours to produce towels for evaluation wherein the amount of the treating alkoxysilane based on the towel was changed in the range of 2.5 to 25 % by weight. The resulting towels were evaluated for quick-drying properties and water absorption by methods shown below. The results are shown in Table 3.

<Method of Pretreatment of the Cotton Towels>

Cotton towels (T. W220, white, manufactured by Takei Towel Co., Ltd.) were washed repeatedly 10 times with a clothing detergent (Liquid Attack, manufactured by Kao Corporation) in an automatic washing machine (Hitachi Automatic Washing Machine KW-5026 "Shizuka Gozen") (37 g detergent, 57 L tap water was used, washing for 5 minutes → rinsing once with running water → dehydration for 3 minutes) . After dehydration in the final round of treatment was finished, the towels were hung and air-dried in a room to give pretreated towels. The weight of each of the pretreated towels was 70 g.

<Method of Evaluation of Quick-Drying Properties>

The towels for evaluation were washed in an automatic washing machine (Hitachi Automatic Washing Machine KW-5026 "Shizuka Gozen") (30 g detergent, 45 L tap water was used, washing for 5 minutes → rinsing once with running water → dehydration for 3 minutes), and after dehydration was finished, the towels were hung and air-dried at a constant temperature of 20°C under 65% RH until their weight became constant. The water content (%) with time was determined according to the following equation (I). The time in which the water content became 10% after initiation of drying was used as an indicator of quick-drying properties.

Water content (%) = {weight (g) of towel just after dehydration - weight (g) of towel reaching a constant weight}/weight (g) of towel reaching a constant weight × 100 (I)

<Method of Evaluation of Water Absorption (Bireck Method)>

A plain-weave portion of the towel was cut into a rectangular strip with a dimension of 2 cmx25 cm, and this strip cloth was suspended in the vertical direction by fixing its upper edge, and after the lower edge, 1 cm, was dipped in water at 20°C, the height of water absorbed was observed with time (1 minute, 3 minutes, 5 minutes and 10 minutes) with the naked eye and recorded in the unit of mm. This measurement was carried out in a room at constant temperature/humidity (20°C/65% RH) .

Examples 12 and 13

Cotton towels pretreated in the same manner as in Example 6 were dipped for 60 minutes in the same coating solutions C10. and C11 as in Examples 10 and 11 and then dried at 80°C for 12 hours. The towels were washed under the same washing conditions as in the above method of evaluating quick-drying properties, dehydrated and dried. The dipping/washing/drying treatment was carried out 10 times to produce accumulatively treated towels for evaluation. The amount of the treating alkoxysilane in each treatment was 2.5% by weight based on the towel. The resulting towels were evaluated for their drying properties and water absorption in the same manner as in Example 6. The results are shown in Table 3.

Examples 14 and 15

A nonioriic surfactant-free catalyst solution B2 or B4 was used to prepare treating solutions C12 and C13 having the compositions shown in Table 3. Cotton towels pretreated in the same manner as in Example 6 were dipped in this coating solution for 60 minutes and then dried at 80˚C for 12 hours, whereby the towels for evaluation in which the amount of the treating alkoxysilane based on the towel was 25 % by weight were produced. The resulting towels were evaluated for their drying properties and water absorption in the same manner as in Example 6. The results are shown in Table 3.

Comparative Example 3

Towels treated in the same manner as in Example 6 and not treated with the coating solution of the present invention were used as towels for evaluation and evaluated for their quick-drying properties and water absorption in the same manner as in Example 6. The results are shown in Table 3.

Table 3

| | | Example 6 | Example 7 | Example 8 | Example 9 | Example 10 | Example 11 | Example 12 | Example 13 | Example 14 | Example 15 | Comparative example 3 |
|---|---|---|---|---|---|---|---|---|---|---|---|---|
| Treating solution | | C6 | C7 | C8 | C9 | C10 | C11 | C10 | C11 | C12 | C13 | Not treated |
| Component (a) | Methyltriethoxysilane (g) | 87.3 | 62.3 | 22.1 | 15.7 | 3.5 | 2.5 | 3.5 | 2.5 | 17.4 | 12.2 | |
| | Dimethyldiethoxysilane (g) | - | 26.8 | - | 6.7 | - | 1.1 | - | 1.1 | - | 52 | |
| Treating solution | Kind | B1 | B3 | B1 | B3 | B1 | B3 | B1 | B3 | B2 | B4 | |
| | Amount (g) | 261.8 | 2683 | 66.2 | 67.1 | 10.6 | 10.6 | 10.6 | 10.6 | 52.3 | 52 3 | |
| Diluent water (g) | | 523.5 | 537.0 | 529.6 | 536.5 | 211.2 | 211.0 | 211.2 | 211.0 | 104.6 | 104.6 | |
| weight of treating solution (g) | | 872.6 | 894.4 | 617.9 | 625.9 | 225.3 | 225.1 | 225 3 | 225.1 | 174.3 | 174.3 | |
| Composition | Component (a) (%) | 10.01 | 6.97 | 3.57 | 2.50 | 1.56 | 1.09 | 1.56 | 1.09 | 9.99 | 7.00 | |
| | Adipic acid (%) | 0.30 | 0.03 | 0.11 | 0.01 | 0.05 | 0.005 | 0.05 | 0.005 | 0.30 | 0.03 | |
| | Emulgen 108 (%) | 2.00 | 2.00 | 0.71 | 0.71 | 0.31 | 0.31 | 0 31 | 0.31 | - | - | |
| | Water (%) | 87.70 | 88.01 | 95 61 | 95.70 | 98.08 | 98.12 | 98.08 | 98 12 | 89.71 | 89 98 | |
| Methyltriethoxysilane/ dimethyldiethoxysilane (weight ratio) | | 10/0 | 7/3 | 10/0 | 7/3 | 10/0 | 7/3 | 10/0 | 7/3 | 10/0 | 7/3 | |
| Silicon content (%) | | 1.6 | 1.7 | 0.6 | 0.6 | 0.2 | 0.3 | 0.2 | 0.3 | 1.6 | 1.7 | |
| Water/component (a) (molar ratio) | | 86.8 | 82.5 | 265.2 | 250.2 | 621.8 | 586.8 | 621.8 | 586.8 | 89.0 | 84.2 | |
| pH (20˚C) | | 3.0 | 3.6 | 3.2 | 3.7 | 3.3 | 3.8 | 3.3 | 3.8 | 2.7 | 3.1 | |
| Treated fibers | Kind | Cotton towel | Cotton towel | Cotton towel | Cotton towel | Cotton towel | Cotton towel | Cotton towel | Cotton towel | Cotton towel | Cotton towel | Cotton towel |
| | Fiber weight (g) | 349.0 | 357.7 | 353.1 | 357.7 | 140.8 | 140 7 | 140.8 | 1407 | 69 7 | 69 7 | 68.9 |
| | Treatment amount (%) | 25 | 25 | 6 | 6 | 2.5 | 2.5 | 2.5 | 2.5 | 25 | 25 | - |
| | Increase in weight after treatment (%) | 7.0 | 7.8 | 0.8 | 0.3 | 0.6 | 0.4 | 5.7 | 5.2 | 7.8 | 69 | - |

(continued)

| | | Example 6 | Example 7 | Example 8 | Example 9 | Example 10 | Example 11 | Example 12 | Example 13 | Example 14 | Example 15 | Comparative example 3 |
|---|---|---|---|---|---|---|---|---|---|---|---|---|
| Evaluation result of quick-drying | Water content just after washing [%] | 49 | 52 | 77 | 68 | 82 | 80 | 54 | 55 | 66 | 62 | 92 |
| | Time for 10% drying [hours] | 35 | 3.6 | 3.8 | 36 | 4.8 | 4.8 | 37 | 3.7 | 3.9 | 3.8 | 6.5 |
| Evaluation result of water absorption | water absorption [cm] (after I minutes) | 2.3 | 5.9 | 4.8 | 5.4 | 5.9 | 5.6 | 25 | 6.2 | 4.0 | 6.0 | 5.9 |
| | water absorption [cm] (after 3 minutes) | 4.0 | 8.7 | 8.2 | 8.3 | 8.4 | 8.6 | 40 | 8.9 | 4.8 | 8.9 | 9.0 |
| | water absorption [cm] (after 5 minutes) | 5.6 | 10.4 | 9.4 | 10.4 | 10.2 | 10.1 | 52 | 10.2 | 6.1 | 10.5 | 10.7 |
| | water absorption [cm] (after 10 minutes) | 7.0 | 12.6 | 121 | 12.5 | 12.3 | 12.8 | 7.2 | 12.1 | 10.4 | 12.8 | 13.0 |

As is evident from Table 3, the towels treated with the treating solution of the present invention had a lower water content upon dehydration after washing, and the time for reduction of the water content to 10% was shorter. The towels using a combination of alkyltrialkoxysilane and dialkyldialkoxysilane had high water absorption similar to the untreated towels, thus indicating that they have excellent water absorption. Further, the towels were made excellent in water absorption and quick-drying properties by using a nonionic surfactant in preparing the treating solution.

Examples 16 to 18

The same treating solutions C7, C13 and C11 as in Examples 7, 15 and 11 were prepared, and cotton towels pretreated in the same manner as in Example 6 were dipped therein for 60 minutes and then dried at 80˚C for 12 hours to produce treated towels for revaluation. The amount of the treating alkoxysilane based on the towel was 25 % by weight in Examples 16 and 17, or 2.5 % by weight in Example 18.

The resulting towels were evaluated for their softness by a method shown below and evaluated for water absorption in the same manner as in Example 6. The results are shown in Table 4.

<Method of Evaluation of Softness>

Towels were washed once with a commercial clothing detergent (Liquid Attack, manufactured by Kao Corporation) in an automatic washing machine (Hitachi Automatic Washing Machine KW-5026 "Shizuka Gozen") (37 g detergent, 57 L tap water was used, washing for 5 minutes → rinsing once with running water → dehydration for 3 minutes). The washed towels were air-dried in a room and left for 1 day in a room at constant temperature/humidity (20˚C/65% RH). Thereafter, the towels were evaluated sensorially in triplicate for softness to the touch by a panel of 5 persons, and the average softness was determined. The untreated towels are towels for evaluation in Comparative Example 4 below.

Pointe -3: The treated towel is evidently harder than the untreated towel.
Pointe -2: The treated towel is somewhat harder than the untreated towel.
Point -1 : The treated towel is slightly harder than the untreated towel.
Point 0: The treated towel is identical in hardness with the untreated towel.
Point 1: The treated towel is slightly softer than the untreated towel.
Point 2 : The treated towel is somewhat softer than the untreated towel.
Point 3: The treated towel is evidently softer than the untreated towel.

Comparative Example 4

Towels treated in the same manner as in Example 6 and not treated with the coating solution of the present inventions were used as towels for evaluation, and evaluated for their softness and water absorption in the same manner as in Example 16. The results are shown in Table 4.

Table 4

| | | Example 16 | Example 17 | Example 18 | Comparative example 4 |
|---|---|---|---|---|---|
| Treating solution | Kind | C7 | C13 | C11 | No treated |
| | weight (g) | 894.4 | 174.3 | 225.1 | |
| Treated fibers | Kind | cotton towel | cotton towel | cotton towel | cotton towel |
| | fiber weight (g) | 357.7 | 69.7 | 140.7 | 68.9 |
| | Treatment amount (%) | 25 | 25 | 2.5 | - |
| | Increase in weight after treatment (%) | 7.8 | 6.9 | 0.4 | - |
| Evaluation result of softness | Sensory evaluation point | 2.1 | 2.6 | 2.4 | 0.0 |

(continued)

| | | Example 16 | Example 17 | Example 18 | Comparative example 4 |
|---|---|---|---|---|---|
| Evaluation result of water absorption | Water absorption [cm] (after 1 minute) | 5.9 | 6.0 | 5.6 | 5.9 |
| | Water absorption [cm] (After 3 minutes) | 8.7 | 8.9 | 8.6 | 9.0 |
| | Water absorption [cm] (after 5 minutes) | 10.4 | 10.5 | 10.1 | 10.7 |
| | Water absorption [cm] (after 10 minutes) | 12.6 | 12.8 | 12.8 | 13.0 |

As is evident from Table 4, the towels treated with the treating solution of the present invention had a sufficiently recognizable softer touch than the untreated towels. The treated towels had the same water absorption as the untreated towels, and the fibers could be endowed with softness while maintaining feeling.

Examples 19 to 23

According to the method of preparation of the treating solution in Example 2, treating solutions C7 and C14 to C16 having the compositions shown in Table 5 were prepared. A wool sweater (ram crew neck sweater, gray, manufactured by UNIQLO), silk, rayon tow, hemp, and acetate tow (all of which are commercially available) were used in place of the cotton towels, and these fibers were dipped in the treating solutions for 60 minutes such that the amount of the treating alkoxysilane based on the fibers became 25 % by weight, and then the fibers were dried at 80˚C for 12 hours to produce treated fibers. The resulting fibers were evaluated for their softness in the same manner as in Example 16. The results are shown in Table 5.

Table 5

| | | Example 19 | Example 20 | Example 21 | Example 22 | Example 23 |
|---|---|---|---|---|---|---|
| Treating solution | | C7 | C14 | C15 | C15 | C16 |
| component (a) | Methyltriethoxysilane (g) | 59.7 | 0.8 | 9.3 | 9.3 | 11.7 |
| | Dimethyldiethoxysilene (g) | 25.7 | 0.3 | 4.0 | 4.0 | 5.0 |
| Catalyst solution | kind | B3 | B3 | B3 | B3 | B3 |
| | Amount (g) | 257.0 | 3.3 | 39.7 | 39.7 | 50.0 |
| Diluent water (g) | | 514.4 | 2.2 | 53.0 | 53.0 | 233.3 |
| Weight of treating solution (g) | | 856.7 | 6.5 | 105.9 | 105.9 | 300.0 |
| Composition | component (a) (%) | 6.97 | 11.67 | 8.75 | 8.75 | 3.89 |
| | Adipic acid (%) | 0.03 | 0.05 | 0.04 | 0.04 | 0.02 |
| | Emulgen 108 (%) | 2.00 | 3.33 | 2.50 | 2.50 | 1.11 |
| | water (%) | 88.01 | 79.95 | 84.96 | 84.96 | 93.32 |
| Methyltriethoxysilane/ dimethyldiethoxysilane (weight ratio) | | 7/3 | 7/3 | 7/3 | 7/3 | 7/3 |
| Silicon content (%) | | 1.7. | 2.8 | 2.1 | 2.1 | 0.9 |

(continued)

| | | Example 19 | Example 20 | Example 21 | Example 22 | Example 23 |
|---|---|---|---|---|---|---|
| Water/component(a) (molar ratio) | | 82.5 | 44.8 | 63.5 | 63.5 | 156.9 |
| pH (20˚C) | | 3.6 | 3.6 | 3.6 | 3.6 | 3.6 |
| Treated fiber | kind | wool sweater | Silk | Rayon tow | hemp | Acetate tow |
| | fiber weight (g) | 340.9 | 4.4 | 53.0 | 53.0 | 66.7 |
| | Treatment amount (%) | 25 | 25 | 25 | 25 | 25 |
| Evaluation result of softness | Sensory evaluation point | 2.2 | 0.5 | 3.0 | 0.5 | 1.7 |

As is evident from Table 5, the fibers had a sufficiently recognizable softer touch than the untreated fibers.

Examples 24 to 27

Cotton towels pretreated in the same manner as in Example 6 were dipped for 60 minutes in the same treating solutions C6 to C9 as in Examples 6 to 9 and dried at 80˚C for 12 hours, to produce towels for evaluation. The amount of the treating alkoxysilane based on the towel was 25 % by weight or 6 % by weight. The resulting treated towels were evaluated for prevention of removal of down by the following method. The results are shown in Table 6.

<Method of Evaluating Prevention of Removal of Down>

Five towels were dried for 3 hours in a tumbler-type drying machine (dehumidification-type electric clothing drying machine NH-D502, manufactured by Matsushita Electric Industrial Co., Ltd.), and this procedure was repeated 10 times. From the amount of down remaining on a filter of the drying machine, the degree of down removal was determined according to the following equation:

$$\text{Degree of down removal (\%)} = \text{amount of down remaining on a filter of the drying machine} / \text{weight of towels before drying} \times 100$$

Comparative Example 5

Towels pre-treated in the same manner as in Example 6 and not treated with the coating solution of the present invention were used as towels for evaluation, and evaluated for prevention of removal of down in the same manner as in Example 24. The results are shown in Table 6.

Table 6

| | | Example 24 | Example 25 | Example 26 | Example 27 | Comparative example 5 |
|---|---|---|---|---|---|---|
| Treating solution | kind | C6 | C7 | C8 | C9 | Not treated |
| | Amount (g) | 872.6 | 894.4 | 617.9 | 625.9 | |
| Treated fibers | kind | cotton towel | cotton towel | cotton towel | cotton towel | cotton towel |
| | Fiber weight '(g) | 349.0 | 357.7 | 353.1 | 357.7 | 68.9 |
| | Treatment amount (%) | 25 | 25 | 6.0 | 6.0 | - |
| | Increase in weight after treatment (%) | 7.0 | 7.8 | 0.8 | 0.3 | - |

(continued)

| | | Example 24 | Example 25 | Example 26 | Example 27 | Comparative example 5 |
|---|---|---|---|---|---|---|
| Evaluation result of down removal | Degree of down removal | 0.20 | 0.19 | 0.22 | 0.21 | 0.28 |

Examples 28 and 29

The same wool jerseys as in Example 3 were dipped for 60 minutes in the same coating solutions C3 and C4 as in Examples 3 and 4, and then dried at 80˚C for 12 hours to produce wool jerseys for evaluation. The amount of the treating alkoxysilane based on the wool jersey was 25 % by weight. The treated wool jerseys were evaluated for their wear resistance by the following method. The results are shown in Table 7.

<Method of Evaluating Wear Resistance>

The wool jersey cut in a size of 1.3 cm in width and 19.5 cm in length was wound around a rotating portion of an Acron wear testing machine (for JIS tire rubber) and examined in a wear test under a loading of 4.5 kg at an inclined angle of 5˚ on a truck wheel A36-P5-V, 3000 revolutions, at a rate of 75 rpm, and damage to the portion of the cloth contacting with a whetstone was evaluated under the following criteria:

⊙: Frayed spots (fiber cutting) are less than 10%.
○: Frayed spots (fiber cutting) are 10 to less than 50%.
× : Frayed spots (fiber cutting) are 50% or more.

Comparative Example 6

The same wood jerseys as in Example 3 and not treated with the coating solution of the present invention were used as wool jerseys for evaluation, and evaluated for their wear resistance in the same manner as in Example 28. The results are shown in Table 7.

Table 7

| | | Example 28 | Example 29 | Comparative example 6 |
|---|---|---|---|---|
| Treating solution | Kind | C3 | C4 | Not treated |
| | Weight (g) | 12.4 | 10.8 | |
| Treating fibers | Kind | wool jersey | wool jersey | wool jersey |
| | Fiber weight (g) | 12.4 | 10.8 | 12.1 |
| | Treatment amount (96) | 25 | 25 | - |
| | Increase in weight after treatment (%) | 8.4 | 11.7 | - |
| Evaluation result of wear resistance | Wear test (visual check) | ⊚ | ○ | × |

As is evident from the results in Tables 6 and 7, it was shown that the fibers treated with the treating solution of the present invention, as compared with the untreated fibers, have less generation of down in treatment in the drying machine, improve wear resistance with a whetstone, and increase toughness.

## Claims

1. A fiber-treating agent comprising an alkoxysilane (a), an organic acid (b) and water (c), wherein 50% or more by weight of the component (a) is an alkoxysilane represented by the following formula (1) (referred to hereinafter as

alkoxysilane (1)):

$$R^1_pSi(OR^2)_{4-p} \qquad (1)$$

wherein $R^1$ represents a C1 to C6 linear or branched alkyl group, a phenyl group or a C2 to C6 linear or branched alkenyl group, $R^2$ represents a C1 to C6 linear or branched alkyl group, $R^1_S$ whose number is p may be the same as or different from one another, $R^2$s whose number is (4-p) may be the same as or different from one another and p is an integer of 1 to 3, and the number of moles of the component (c) is 3 times or more as large as that of the component (a) and wherein the amount of component (c) is 30 to 99.9% by weight of the fiber-treating agent.

**2.** The fiber-treating agent according to claim 1, comprising a first agent containing the alkoxysilane (a) wherein 50% or more by weight of the component (a) is the alkoxysilane (1) and a second agent with a pH value of 2 to 5 at 20˚C containing the organic acid (b) and water (c).

**3.** The fiber-treating agent according to claim 1 or 2 having a pH value of 2 to 5 at 20˚C.

**4.** The fiber-treating agent according to claim 3, which comprises a silanol compound (4) formed by hydrolysis of the alkoxysilane (1), the organic acid (b) and water (c),
the silanol compound (4) being represented by the following formula (4) (referred to hereinafter as silanol compound (4)) :

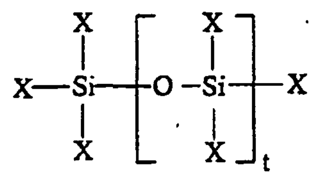

(4)

wherein X is a group represented by $R^1$, $OR^2$ or OH, t is an integer of 0 to 2, X's whose number is (2t + 4) may be the same as or different from one another, and at least one of X's is OH, and $R^1$ and $R^2$ have the same meanings as defined in claim 1.

**5.** The fiber-treating agent according to any of claims 1 to 4, which further comprises a surfactant (d).

**6.** The fiber-treating agent according to any of claims 1 to 5, wherein the component (a) comprises a trialkoxysilane (a1) represented by the formula (2) and a dialkoxysilane (a2) represented by the formula (3):

$$R^1si(OR^2)_3 \qquad (2)$$

$$R^1_2Si\ (OR^2)_2 \qquad (3)$$

wherein $R^1$ and $R^2$ have the same meanings as defined above.

**7.** The fiber-treating agent according to claim 6,
wherein the trialkoxysilane (al)/dialkoxysilane (a2) ratio by weight is from 9/1 to 1/9.

**8.** The fiber-treating agent according to any of claims 1 to 7, which is a quick-drying-conferring agent.

**9.** The fiber-treating agent according to any of claims 1 to 7, which is a softness-conferring agent.

**10.** The fiber-treating agent according to any of claims 1 to 7, which is a toughness-conferring agent.

**11.** A method of producing the fiber-treating agent according to claim 6 or 7, which comprises steps of mixing the trialkoxysilane (a1), the organic acid (b) and water (c) with one another and then mixing the dialkoxysilane (a2) therewith.

**12.** A method of treating fibers, comprising step (i) of bringing the fiber-treating agent according to any of claims 3 to

10 into contact with fibers to penetrate, into the fibers, a silanol compound (4) formed by hydrolysis of the alkoxysilane (1), and step (ii) of polymerizing the silanol compound (4).

**13.** The method of treating fibers according to claim 12, wherein step (ii) is carried out under heating at 60°C or more.

**14.** The method of treating fibers according to claim 12 or 13, which further comprises step (iii) of washing the fibers with water between steps (i) and (ii).

**15.** Use of the fiber-treating agent according to any of claims 3 to 7 for treating fibers.

## Patentansprüche

**1.** Faserbehandlungsmittel, das ein Alkoxysilan (a), eine organische Säure (b) und Wasser (c) umfasst, wobei 50 Gew. % oder mehr der Komponente (a) ein Alkoxysilan der folgenden Formel (1) ist (nachfolgend als Alkoxysilan (1) bezeichnet):

$$R^1pSi(OR^2)_{4-p} \qquad (1)$$

Worin $R^1$ eine C1 bis C6 lineare oder verzweigte Alkylgruppe, eine Phenylgruppe oder eine C2 bis C6 lineare oder verzweigte Alkenylgruppe ist, $R^2$ eine C1 bis C6 lineare oder verzweigte Alkylgruppe ist, die $R^1$, deren Anzahl p ist, können gleich oder verschieden voneinander sein, die $R^2$ deren Anzahl (4-p) ist können gleich oder verschieden voneinander sein und p ist eine ganze Zahl von 1 bis 3 und die Molanzahl der Komponente (c) ist 3-mal so hoch oder mehr als die der Komponente (a), und worin die Menge der Komponente (c) 30 bis 99,9 Gew.% des Faserbehandlungsmittels ist.

**2.** Faserbehandlungsmittel gemäß Anspruch 1, das ein erstes Mittel umfasst, das das Alkoxysilan (a) enthält, wobei 50 Gew.% oder mehr der Komponente (a) das Alkoxysilan (1) ist, und ein zweites Mittel mit einem pH-Wert von 2 bis 5 bei 20°C umfasst, das die organische Säure (b) und Wasser (c) enthält.

**3.** Faserbehandlungsmittel gemäß Anspruch 1 oder 2 mit einem pH-Wert von 2 bis 5 bei 20°C.

**4.** Faserbehandlungsmittel gemäß Anspruch 3, das eine Silanolverbindung (4) umfasst, die durch Hydrolyse des Alkoxysilans (1), der organischen Säure (b) und des Wassers (c) gebildet ist,
wobei die Silanolverbindung (4) durch die folgende Formel (4) dargestellt ist (im folgenden als Silanolverbindung (4) bezeichnet):

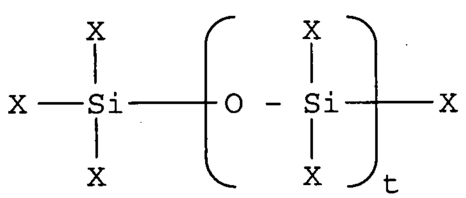

(4)

worin X eine $R^1$-, $OR^2$- oder OH-Gruppe ist, t eine ganze Zahl von 0 bis 2, die X, deren Anzahl (2t + 4) ist, können gleich oder verschieden voneinander sein und mindestens eines der X ist OH und $R^1$ und $R^2$ haben die gleichen Bedeutungen wie in Anspruch 1 definiert.

**5.** Faserbehandlungsmittel gemäß irgendeinem der Ansprüche 1 bis 4, das weiterhin ein Tensid (d) umfasst.

**6.** Faserbehandlungsmittel gemäß irgendeinem der Ansprüche 1 bis 5, worin die Komponente (a) ein Trialkoxysilan (a1) der Formel (2) und ein Dialkoxysilan (a2) in der Formel (3) umfasst:

$$R^1Si(OR^2)_3 \qquad (2)$$

$$R^1_2Si(OR^2)_2 \qquad (3)$$

worin $R^1$ und $R^2$ die gleichen Bedeutungen wie oben definiert haben.

**7.** Faserbehandlungsmittel gemäß Anspruch 6, worin das Trialkoxysilan (a1)/Dialkoxysilan (a2) Gewichtsverhältnis von 9/1 bis 1/9 ist.

**8.** Faserbehandlungsmittel gemäß irgendeinem der Ansprüche 1 bis 7, welches ein Schnelltrocknung-verleihendes Mittel umfasst.

**9.** Faserbehandlungsmittel gemäß irgendeinem der Ansprüche 1 bis 7, welches ein Weichheit verleihendes Mittel umfasst.

**10.** Faserbehandlungsmittel gemäß irgendeinem der Ansprüche 1 bis 7, welches ein Härte-verleihendes Mittel umfasst.

**11.** Verfahren zur Herstellung eines Faserbehandlungsmittels gemäß Anspruch 6 oder 7, welches die Schritte des Mischens des Trialkoxysilans (a1), der organischen Säure (b) und des Wasser (c) miteinander und anschließend das Mischen des Dialkoxysilans (a2) damit umfasst.

**12.** Verfahren zur Behandlung von Fasern, welches den Schritt (i) des in Kontaktbringens des Faserbehandlungsmittels gemäß irgendeinem der Ansprüche 3 bis 10 mit Fasern umfasst, wodurch eine Silanolverbindung (4) in die Fasern eindringt, die durch Hydrolyse des Alkoxysilans (1) gebildet wird, und den Schritt (ii) der Polymerisation der Silanolverbindung (4) umfasst.

**13.** Verfahren zur Behandlung von Fasern gemäß Anspruch 12, wobei Schritt (ii) unter Erwärmen auf 60˚C oder mehr durchgeführt wird.

**14.** Verfahren zur Behandlung von Fasern gemäß Anspruch 12 oder 13, welches weiterhin den Schritt (iii) des Waschens der Fasern mit Wasser zwischen den Schritten (i) und (ii) umfasst.

**15.** Verwendung des Faserbehandlungsmittels gemäß irgendeinem der Ansprüche 3 bis 7 zur Behandlung von Fasern.

## Revendications

**1.** Agent de traitement de fibres comprenant un alcoxysilane (a), un acide organique (b) et de l'eau (c), dans lequel au moins 50 % en poids du constituant (a) est un alcoxysilane représenté par la formule (1) ci-dessous (appelé alcoxysilane (1) dans ce qui suit):

$$R^1_pSi(OR^2)_{4-p} \quad (1)$$

où $R^1$ représente un groupe alkyle en $C_1$-$C_6$ linéaire ou ramifié, un groupe phényle ou un groupe alcényle en $C_2$-$C_6$ linéaire ou ramifié, $R^2$ représente un groupe alkyle en $C_1$-$C_6$ linéaire ou ramifié, les $R^1$ dont le nombre est p peuvent être identiques ou différents les uns des autres, les $R^2$ dont le nombre est (4-p) peuvent être identiques ou différents les uns des autres, et p est un entier de 1 à 3, et le nombre de moles du constituant (c) est au moins 3 fois plus élevé que celui du constituant (a), et la quantité de constituant (c) est de 30 à 99,9 % en poids de l'agent de traitement de fibres.

**2.** Agent de traitement de fibres selon la revendication 1, comprenant un premier agent contenant l'alcoxysilane (a), au moins 50 % en poids du constituant (a) étant l'alcoxysilane (1), et un deuxième agent ayant une valeur de pH de 2 à 5 à 20˚C, contenant l'acide organique (b) et de l'eau (c).

**3.** Agent de traitement de fibres selon la revendication 1 ou 2, ayant une valeur de pH de 2 à 5 à 20˚C.

**4.** Agent de traitement de fibres selon la revendication 3, qui comprend un composé silanol (4) formé par hydrolyse de l'alcoxysilane (1), l'acide organique (b) et l'eau (c),
le composé silanol (4) étant représenté par la formule (4) ci-dessous (appelé composé silanol (4) dans ce qui suit):

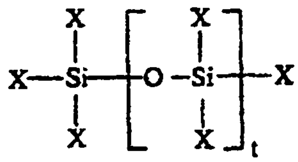 (4)

où X est un groupe représenté par $R^1$, $OR^2$ ou OH, t est un entier de 0 à 2, les X dont le nombre est (2t+4) peuvent être identiques ou différents les uns des autres, et au moins l'un des X est OH, et $R^1$ et $R^2$ sont tels que définis dans la revendication 1.

**5.** Agent de traitement de fibres selon l'une quelconque des revendications 1 à 4, qui comprend en outre un agent tensioactif (d).

**6.** Agent de traitement de fibres selon l'une quelconque des revendications 1 à 5, dans lequel le constituant (a) comprend un trialcoxysilane (a1) représenté par la formule (2) et un dialcoxysilane (a2) représenté par la formule (3):

$R^1Si(OR^2)_3$ (2)

$R^1{}_2Si(OR^2)_2$ (3)

où $R^1$ et $R^2$ sont tels que définis ci-dessus.

**7.** Agent de traitement de fibres selon la revendication 6, dans lequel le rapport en poids trialcoxysilane (a1) / dialcoxysilane (a2) est de 9/1 à 1/9.

**8.** Agent de traitement de fibres selon l'une quelconque des revendications 1 à 7, qui est un agent conférant un séchage rapide.

**9.** Agent de traitement de fibres selon l'une quelconque des revendications 1 à 7, qui est un agent conférant une souplesse.

**10.** Agent de traitement de fibres selon l'une quelconque des revendications 1 à 7, qui est un agent conférant une dureté.

**11.** Procédé de fabrication de l'agent de traitement de fibres selon la revendication 6 ou 7, qui comprend les étapes de mélange du trialcoxysilane (a1), de l'acide organique (b) et de l'eau (c) entre eux, puis de mélange du dialcoxysilane (a2) avec ceux-ci.

**12.** Procédé de traitement de fibres, comprenant l'étape (i) de mise en contact de l'agent de traitement de fibres selon l'une quelconque des revendications 3 à 10 avec des fibres pour faire pénétrer dans les fibres un composé silanol (4) formé par hydrolyse de l'alcoxysilane (1), et l'étape (ii) de polymérisation du composé silanol (4).

**13.** Procédé de traitement de fibres selon la revendication 12, dans lequel on effectue l'étape (ii) en chauffant à au moins 60°C.

**14.** Procédé de traitement de fibres selon la revendication 12 ou 13, qui comprend en outre l'étape (iii) de lavage des fibres avec de l'eau entre les étapes (i) et (ii).

**15.** Utilisation de l'agent de traitement de fibres selon l'une quelconque des revendications 3 à 7 pour le traitement de fibres.

Fig.1

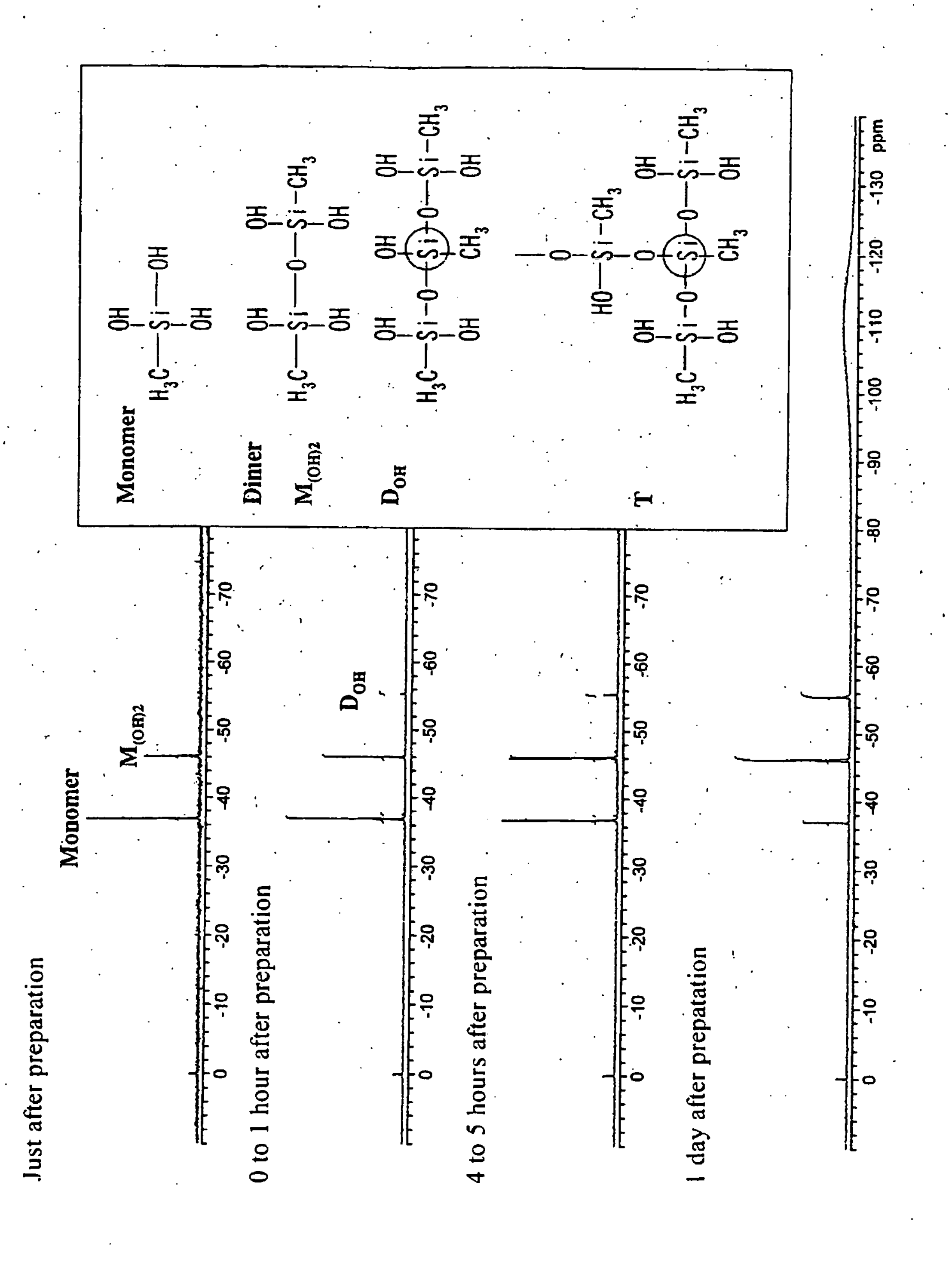
Just after preparation
0 to 1 hour after preparation
4 to 5 hours after preparation
1 day after prepatation
Monomer
M(OH)2
DOH
Dimer
T
ppm
0
-10
-20
-30
-40
-50
-60
-70
-80
-90
-100
-110
-120
-130

Fig.2

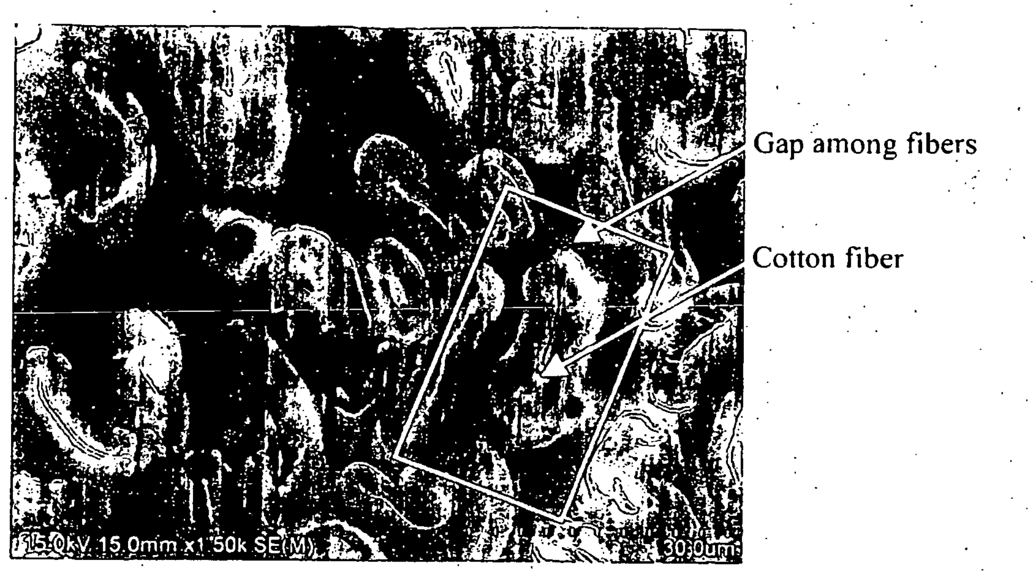
Gap among fibers
Cotton fiber
15.0kV 15.0mm x1.50k SE(M)
30.0um

Fig.3

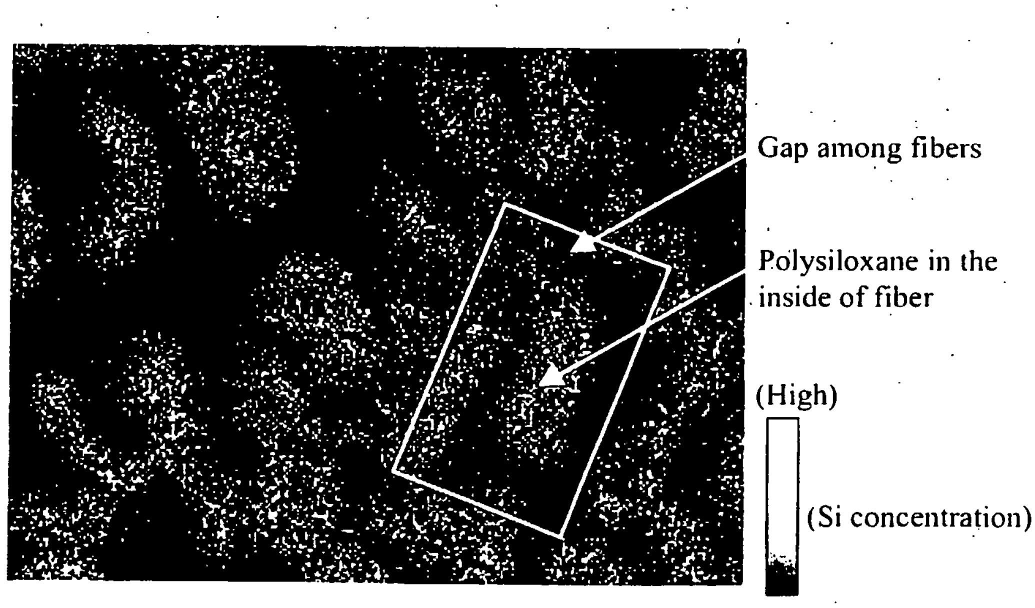
Gap among fibers
Polysiloxane in the inside of fiber
(High)
(Si concentration)

Fig.4

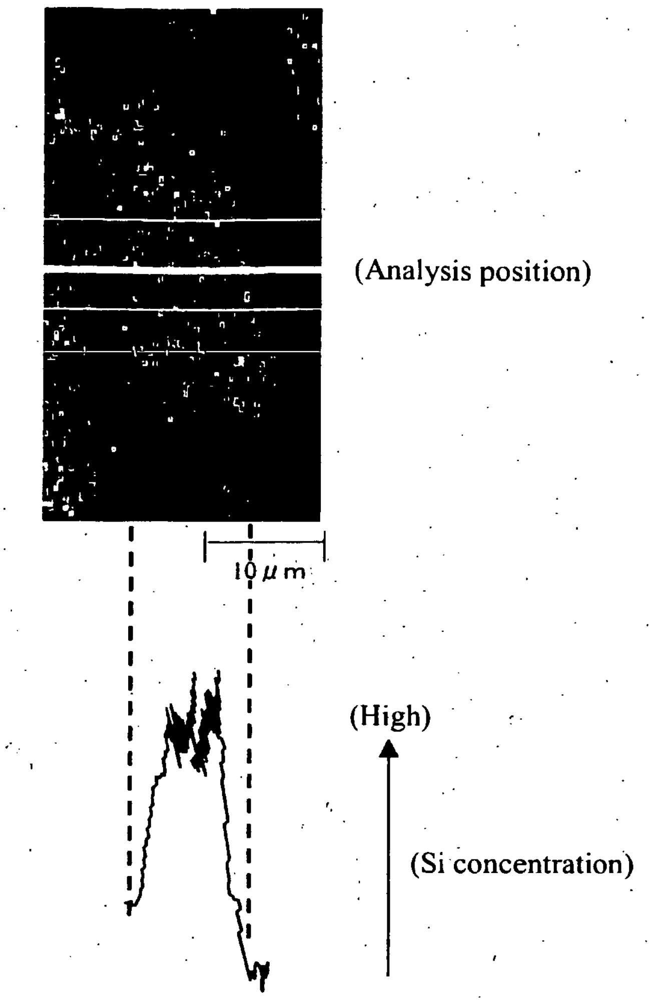
(Analysis position)
10 μm
(High)
(Si concentration)

Fig.5

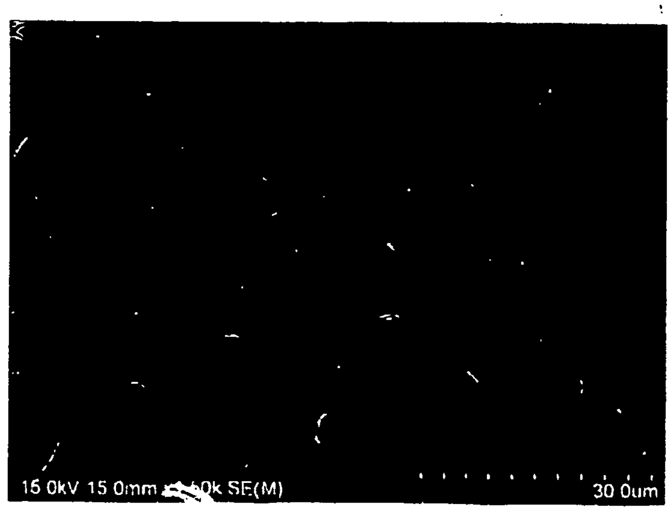
15.0kV 15.0mm
SE(M)
30.0um

Fig.6

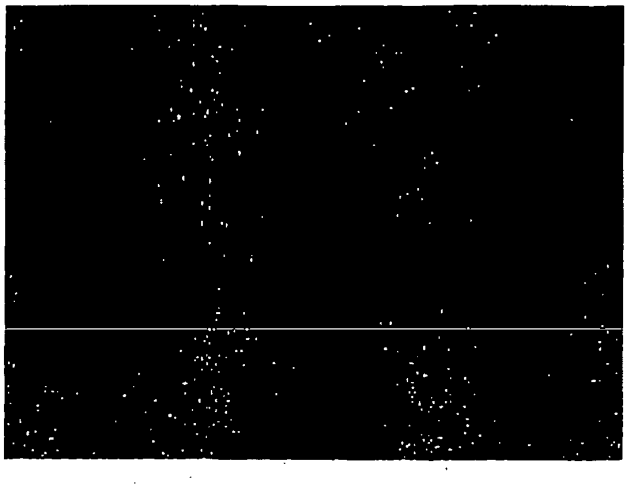

Fig.7

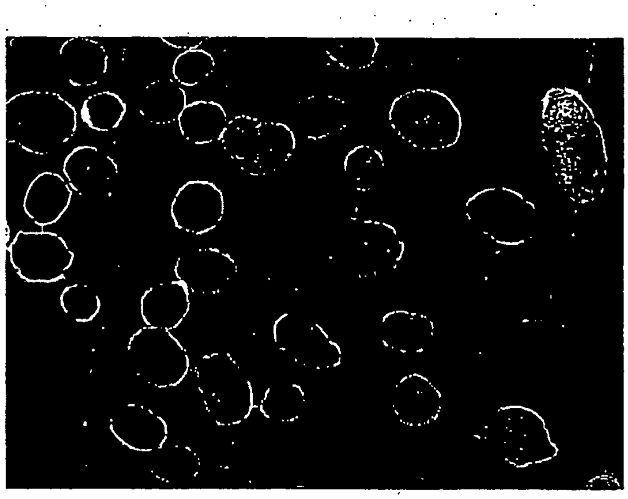

Fig.8

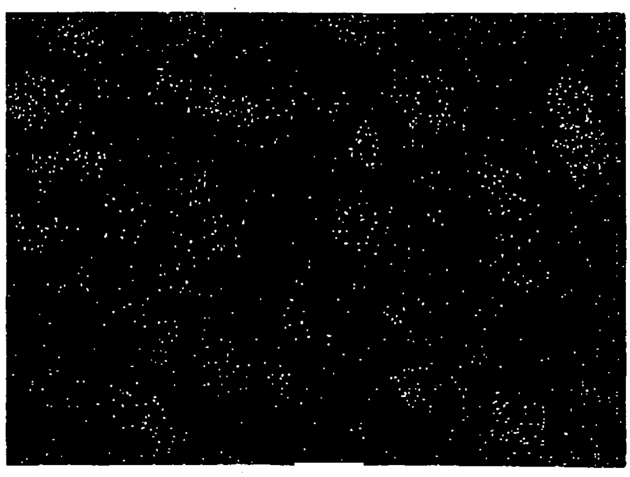

Fig.9

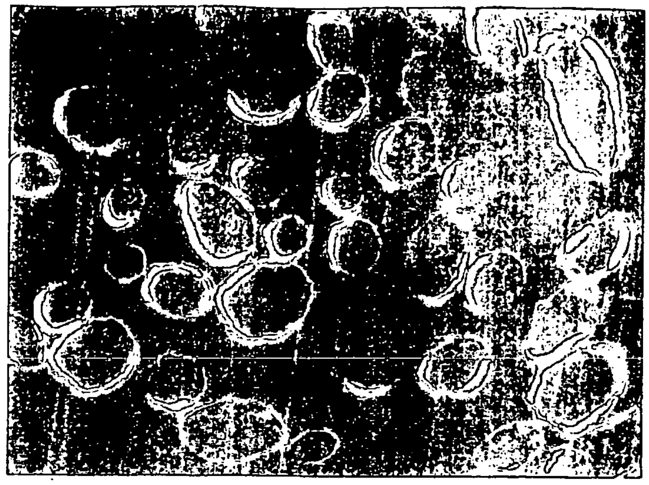

Fig.10

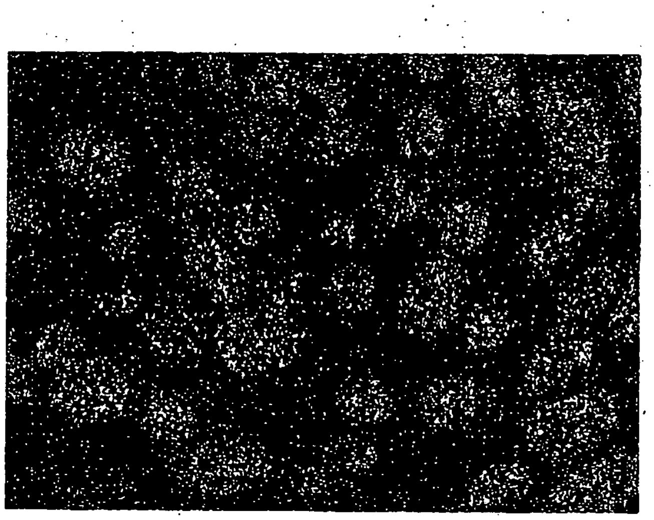

Fig.11

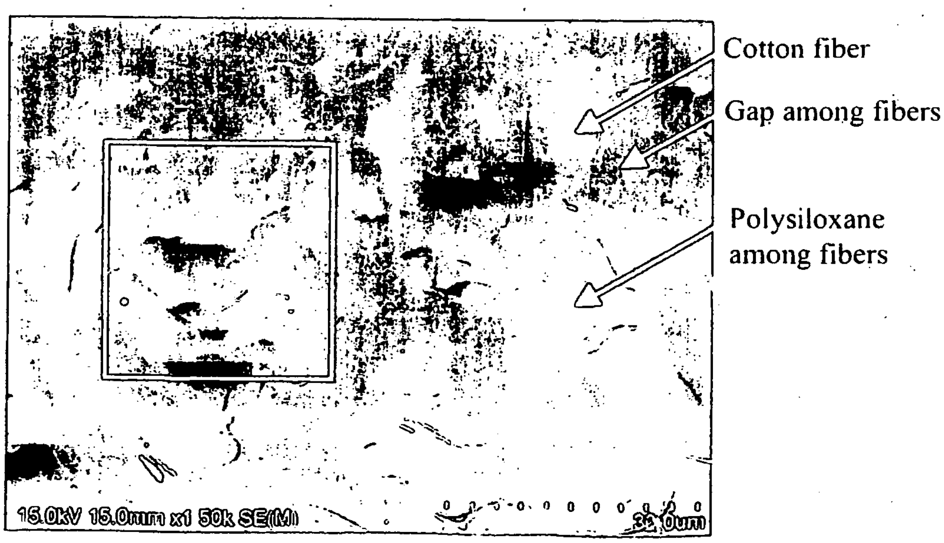

Fig.12

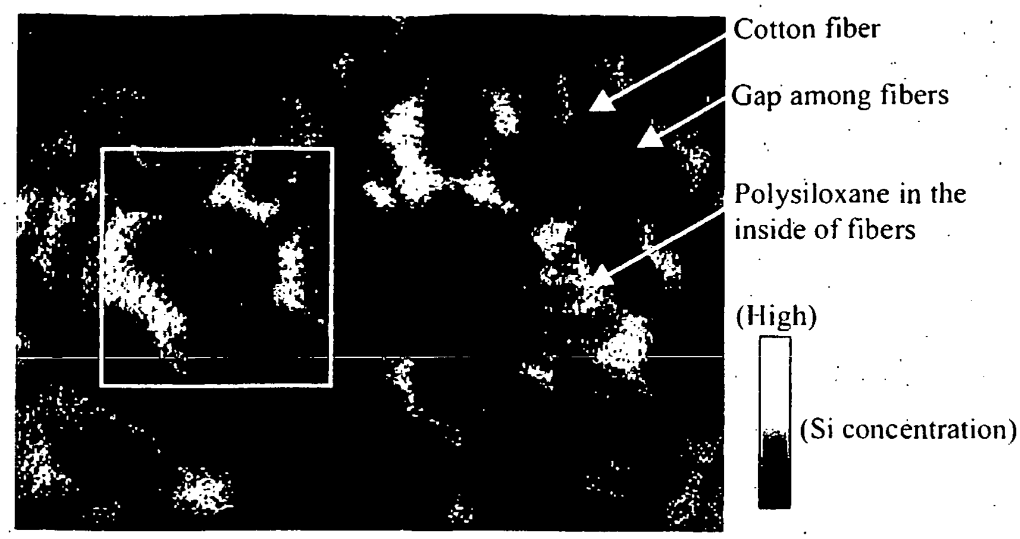
Cotton fiber
Gap among fibers
Polysiloxane in the inside of fibers
(High)
(Si concentration)

Fig.13

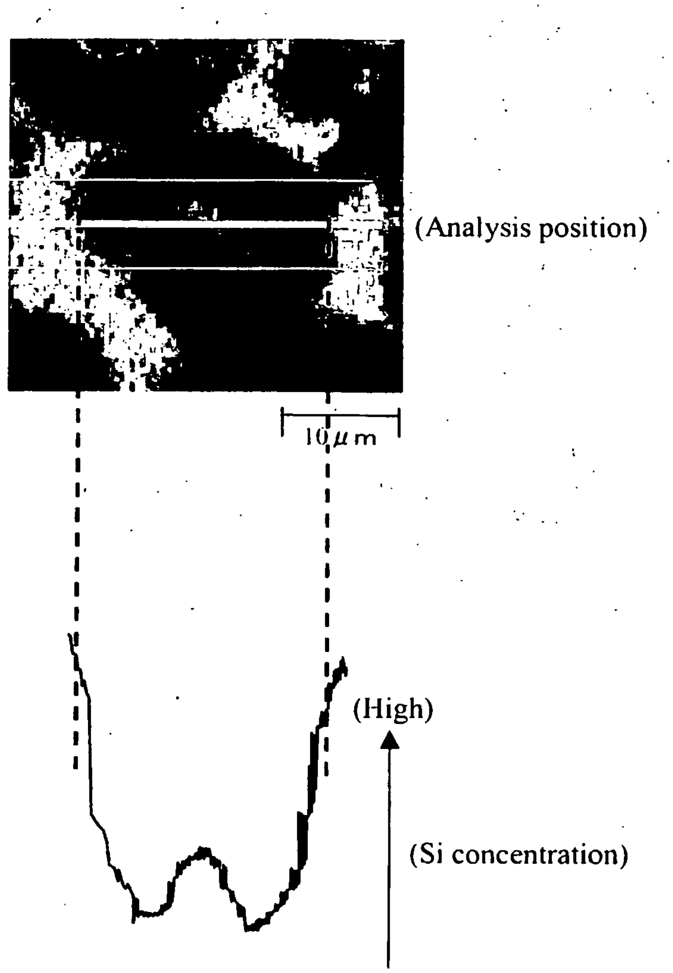
(Analysis position)
10μm
(High)
(Si concentration)

Fig.14

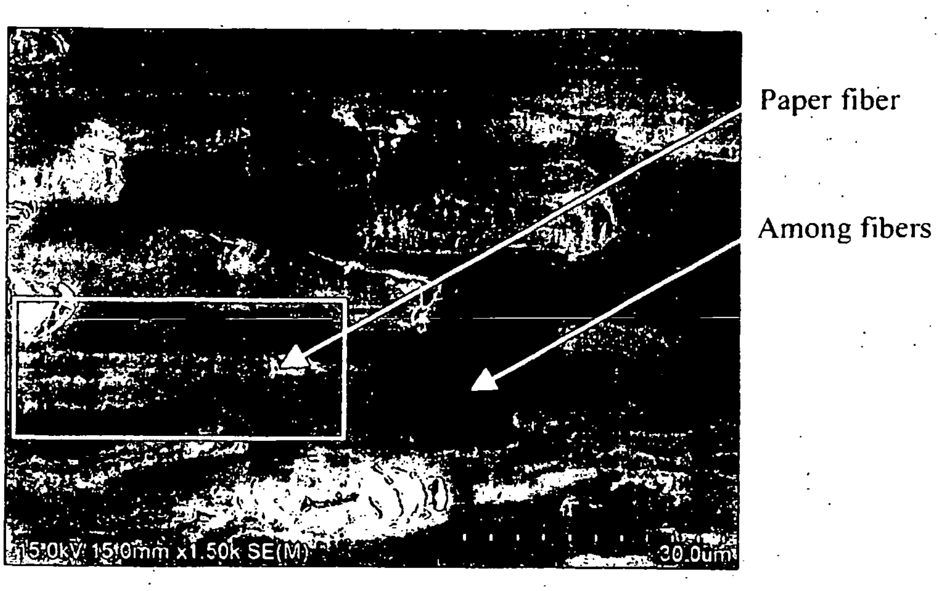
15.0kV 15.0mm x1.50k SE(M)
30.0um
Paper fiber
Among fibers

Fig.15

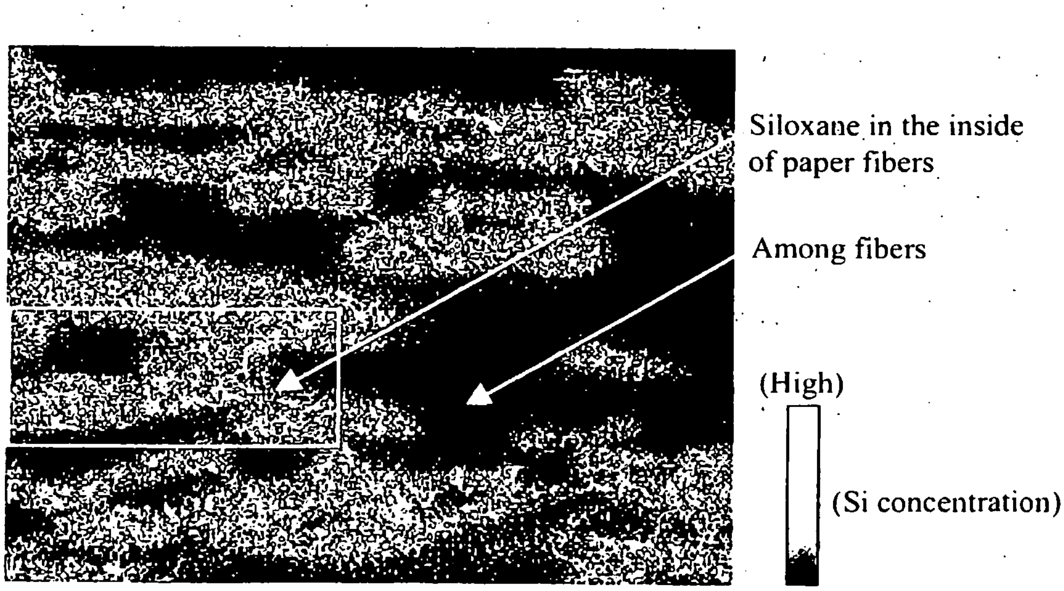
Siloxane in the inside of paper fibers
Among fibers
(High)
(Si concentration)

Fig.16

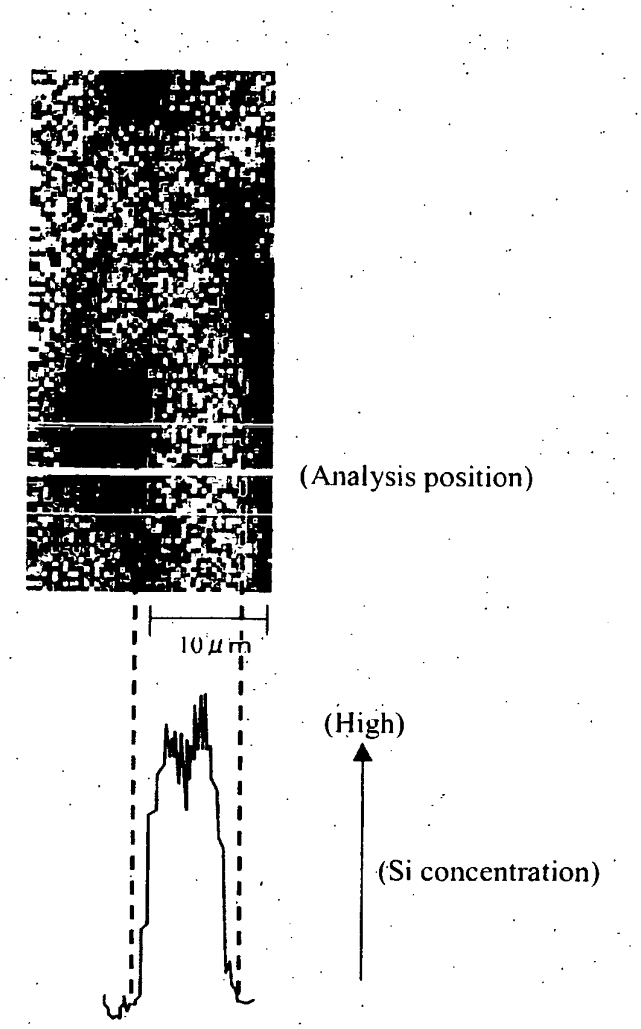
(Analysis position)
10 μm
(High)
(Si concentration)

Fig.17

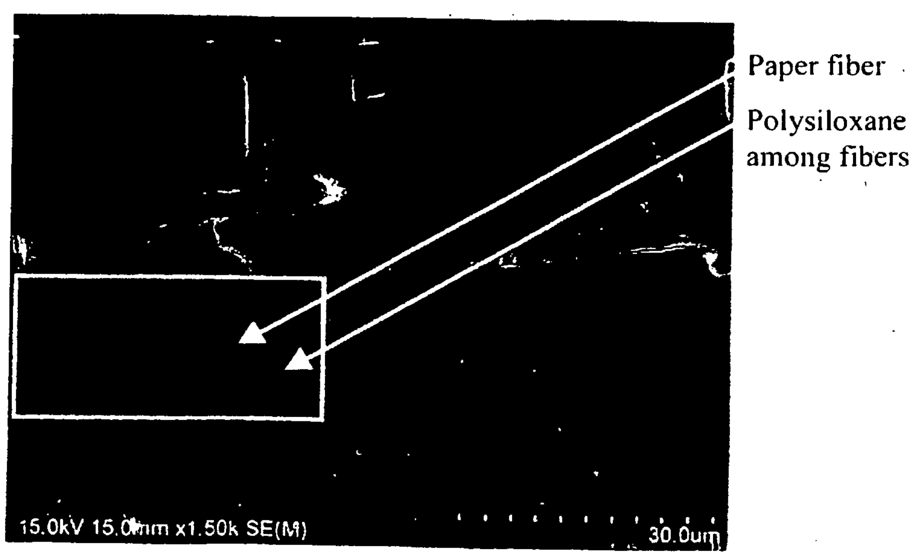
Paper fiber
Polysiloxane among fibers
15.0kV 15.0mm x1.50k SE(M)
30.0um

Fig.18

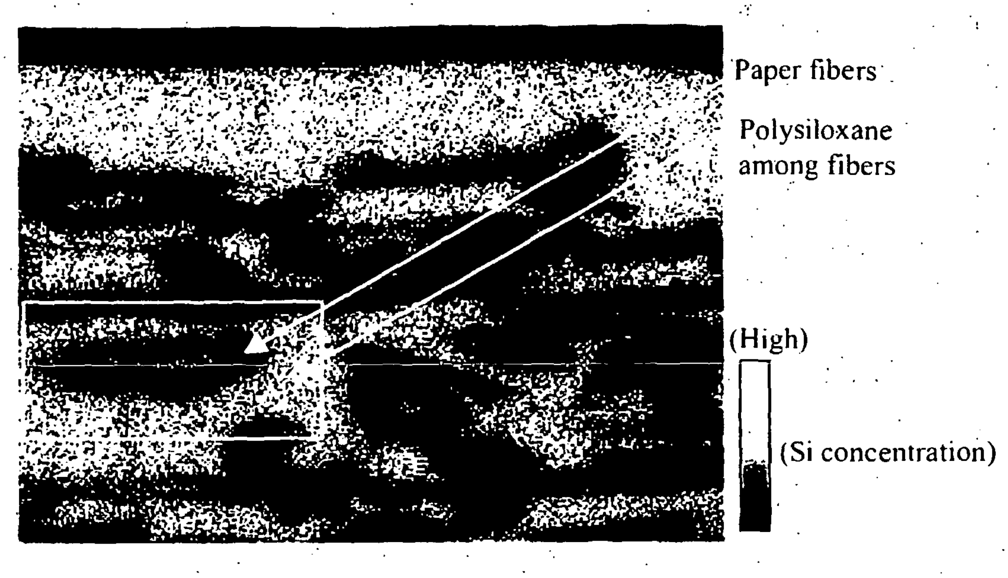
Paper fibers
Polysiloxane among fibers
(High)
(Si concentration)

Fig.19

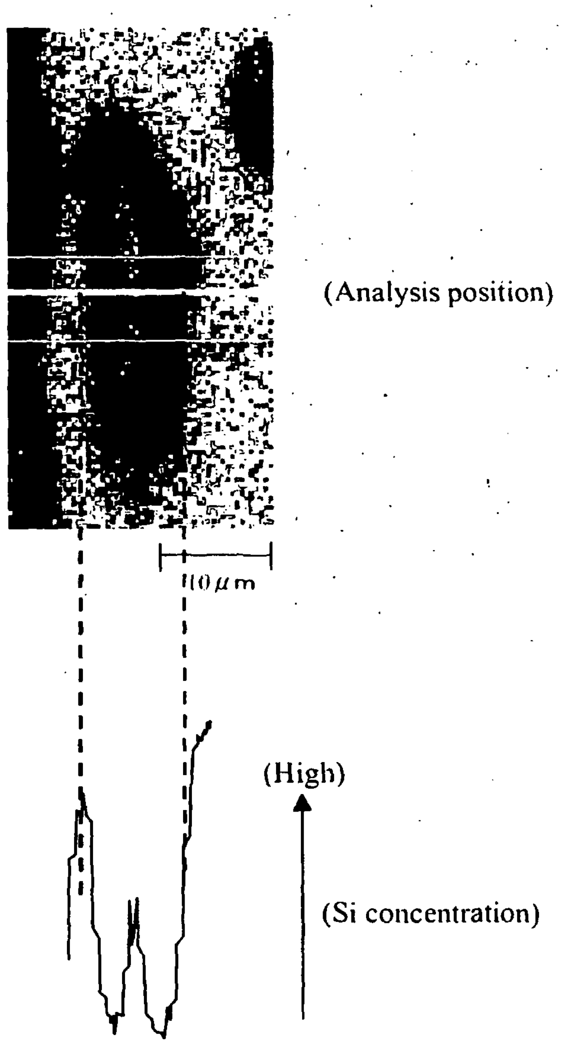
(Analysis position)
10 μm
(High)
(Si concentration)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2003342875 A
- JP 2005089882 A
- JP 2002061094 A
- JP 9249748 A
- JP 2001181599 A
- EP 0535649 A1
- US 4517375 A
- US 4170690 A
- GB 0781488 A
- EP 1179633 A